(19) Europäisches Patentamt / European Patent Office / Office européen des brevets

(11) **EP 4 156 178 B1**

(12) **EUROPÄISCHE PATENTSCHRIFT**

(45) Veröffentlichungstag und Bekanntmachung des Hinweises auf die Patenterteilung:
**08.04.2026 Patentblatt 2026/15**

(21) Anmeldenummer: **21198529.6**

(22) Anmeldetag: **23.09.2021**

(51) Internationale Patentklassifikation (IPC):
**G10L 15/22** *(2006.01)* **A61B 17/00** *(2006.01)*

(52) Gemeinsame Patentklassifikation (CPC):
**G10L 15/22;** A61B 2017/00203; G10L 2015/223

(54) **SPRACHSTEUERUNG EINER MEDIZINISCHEN VORRICHTUNG**

VOICE CONTROL OF A MEDICAL DEVICE

COMMANDE VOCALE D'UN DISPOSITIF MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**29.03.2023 Patentblatt 2023/13**

(73) Patentinhaber: **Siemens Healthineers AG**
**91301 Forchheim (DE)**

(72) Erfinder:
• **ROTHBAUER, Stefan**
**86156 Augsburg (DE)**

• **KUHRT, Sören**
**91056 Erlangen (DE)**
• **KILIAN, Lennart, Dr.**
**82131 Gauting (DE)**

(74) Vertreter: **Siemens Healthineers**
**Patent Attorneys**
**Postfach 22 16 34**
**80506 München (DE)**

(56) Entgegenhaltungen:
EP-A2- 0 201 883      US-A1- 2014 153 747
US-A1- 2014 195 247   US-A1- 2018 218 739
US-A1- 2020 029 795   US-A1- 2020 211 573
US-B1- 9 824 689

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Sprachsteuerung einer medizinischen Vorrichtung durch Verarbeiten eines Audiosignals enthaltend eine auf die Steuerung der Vorrichtung gerichtete Spracheingabe einer Bedienperson. Insbesondere betrifft die Erfindung ein Verfahren zur erstfehlersicheren Sprachsteuerung einer medizinischen Vorrichtung. Die Erfindung betrifft auch ein entsprechendes medizinisches System aufweisend eine medizinische Vorrichtung.

Medizinische Vorrichtungen werden typischerweise zur Behandlung und/oder Untersuchung und/oder Überwachung eines Patienten eingesetzt, wie bspw. bildgebende Modalitäten wie Magnetresonanzvorrichtungen, Computertomographievorrichtungen, PET-Vorrichtungen (Positronen-Emissions-Tomographie-Vorrichtungen) oder Interventions- und/oder Therapievorrichtungen wie Strahlen- bzw. Radiotherapievorrichtungen. Die Behandlung und/oder Untersuchung des Patienten wird typischerweise von einem Bedienpersonal unterstützt.

Vor und während der Durchführung einer Behandlung und/oder Untersuchung eines Patienten mit einer solchen medizinischen Vorrichtung sind üblicherweise verschiedene Einstellungen an der medizinischen Vorrichtung vorzunehmen, wie beispielsweise eine Eingabe von Patientendaten, eine Einstellung verschiedener Geräteparameter und dergleichen. Diese Schritte werden von dem Bedienpersonal durchgeführt, wobei die Vornahme der Einstellungen der medizinischen Vorrichtung typischerweise über eine an der Vorrichtung bereitgestellte physische Benutzerschnittstelle erfolgt, in welche eine Bedienperson Eingaben vornehmen kann.

Um solche medizinischen Vorrichtungen wirtschaftlich zu betreiben, ist ein reibungsloser Arbeitsbzw. Verfahrensablauf wünschenswert. Insbesondere soll die Vornahme von Einstellungen so einfach wie möglich gestaltet werden. Dazu eignet sich insbesondere eine Sprachsteuerung, bei der eine Bedienperson Steuerbefehle über ein natürliches Sprachsignal an die medizinische Vorrichtung übergibt. Die DE 10 2006 045 719 B4 beschreibt in dieser Hinsicht ein medizinisches System mit Spracheingabevorrichtung, bei dem spezifische Funktionen des Systems mittels einer Sprachsteuerung aktiviert und deaktiviert werden können. Dabei wird ein mit der Spracheingabevorrichtung erfasstes Audiosignal mit einem Sprachanalysemodul verarbeitet, um Sprachbefehle einer Bedienperson zu ermitteln.

Bei der Sprachsteuerung, also der Analyse bzw. der Erkennung einer mittels natürlicher Sprache formulierten Nutzerintention bzw. eines Sprachbefehls kommen bevorzugt Algorithmen der künstlichen Intelligenz, bevorzugt neuronaler Netze zum Einsatz. Diese sind besonders gut geeignet, einen hochdimensionalen Eingangsraum umfassend eine Vielzahl von verschiedenen Sprachsequenzen entsprechend natürlicher Spracheingaben auf einen Zielraum umfassend eine Anzahl definierter Steuerbefehle abzubilden.

Viele medizinische Vorrichtungen müssen für eine Zulassung zudem das Erfordernis der Erstfehlersicherheit bzw. der funktionalen Sicherheit erfüllen, um die Sicherheit von Patient und Bedienpersonal zu jeder Zeit während des zunehmend automatisierten Betriebs der medizinischen Vorrichtung zu gewährleisten. Erstfehlersicherheit bedeutet, dass kein einziger erster Fehler dazu führen kann, dass eine Nutzung der medizinischen Vorrichtung während ihrer Lebensdauer unsicher werden kann.

Ein besonders sicherheitskritischer Steuerbefehl ist bspw. auf das Auslösen/Starten einer Röntgenstrahlung bei der Bilddatenerfassung oder der Strahlentherapie gerichtet. Ein anderes Beispiel eines sicherheitskritischen Steuerbefehls betrifft die (autonome) Verstellbewegung einer medizinischen Vorrichtung oder einer ihrer Komponenten, bspw. ein Roboterarm, im Raum. Nicht autorisierte bzw. nicht bestätigte Strahlungsauslösung bzw. Vorrichtungsbewegung kann direkt das Wohl des Patienten bzw. einer Bedienperson gefährden.

Verfahren, um die unbeabsichtigte Ausführung kritischer Aktionen durch Sprachbefehle zu vermeiden, sind zum Beispiel in US 2014/0195247 A1 und US 9,824,689 B1 beschrieben.

Um Hardware und/oder Steuerungssoftware einer medizinischen Vorrichtung zur Verarbeitung und Umwandlung von mittels beliebiger Benutzerschnittstelle erfasster Steuerbefehle funktional sicher oder erstfehlersicher auszubilden, ist es üblich, eine manuelle Autorisierung eines erkannten Steuerbefehls von einer Bedienperson einzufordern. Ein sogenannter Totmann-Schalter (dead man grip) soll hier als Beispiel dienen. Dieser Schalter/Hebel/Griff muss von einer Bedienperson kontinuierlich betätigt werden, um eine automatische Verstellbewegung an einer medizinischen Vorrichtung zu vollziehen. Die Verstellbewegung stoppt automatisch, wenn die Bedienperson den Totmann-Schalter loslässt.

Alternativ dazu kann die medizinische Vorrichtung zur Absicherung einer Steuerungssoftware ein zweites, redundantes Softwaresystem auf unabhängiger Hardware, d.h. eigenem Prozessor bzw. eigenem Speicher betrieben. Nur wenn das redundante Softwaresystem den initial erkannten Steuerbefehl plausibilisiert, wird der Steuerbefehl tatsächlich von der medizinischen Vorrichtung ausgeführt, ansonsten wird er verworfen.

Im Bereich der Sprachsteuerung mangelt es derzeit noch grundlegend an etablierten und verlässlichen Methoden zum Nachweis einer für eine funktionale oder Erstfehlersicherheit erforderlichen Qualität der Spracherkennungsalgorithmen. Bspw. die Frage nach universellen Kriterien, denen ein Trainingsdatensatz eines erstfehlersicheren (KI-) Spracherkennungsalgorithmus (KI = künstliche Intelligenz) genügen muss oder nach einem allgemeingültigen Robustheitsmaß eines (KI-) Spracherkennungsalgorithmus zur korrekten Klassifizierung einer bspw. durch Verzerrungen oder Hintergrundgeräusche veränderten Spracheingabe sind Gegenstand aktueller Forschung. Auch ein an bestehende Sicherheitsnormen angelehntes Vorgehen zur Verifikation eines erkannten Sprachbefehls ist nicht ausreichend sicher bzw. nicht möglich, denn es fehlen auch hier klassisch definierte Anforderungen/Kriterien für KI-Spracherkennungsalgorithmen, um deren Erfüllung nachzuweisen. Dies wäre zwingende Voraussetzung für eine Zulassung bzw. Zertifizierung der (KI-)Spracherkennungsalgorithmen. Daneben würde das Verifizieren eines erkannten Sprachbefehls mittels eines identischen, redundanten Spracherkennungsalgorithmus, der im Zweifel den Erstfehler wiederholt, keine Erstfehlersicherheit gewährleisten.

Infolgedessen kommt eine Sprachsteuerung von medizinischen Vorrichtungen bislang nur außerhalb sicherheitskritischer Anwendungen zum Einsatz.

Aufgabe der vorliegenden Erfindung ist es, dieses Problem zu lösen und Mittel zur Sprachsteuerung einer medizinischen Vorrichtung bereitzustellen, welches eine verbesserte, da verlässlichere Ermittlung von Sprachbefehlen einer Bedienperson aus einem Audiosignal erlaubt. Insbesondere ist es Aufgabe der vorliegenden Erfindung, Mittel bereitzustellen, die eine Erstfehlersicherheit mittels einer Sprachsteuerung gewährleisten.

Diese Aufgabe wird erfindungsgemäß gelöst durch ein Verfahren zur Sprachsteuerung einer medizinischen Vorrichtung, eine entsprechende Sprachsteuervorrichtung, ein medizinisches System umfassend die Sprachsteuervorrichtung, ein Computerprogrammprodukt sowie ein computerlesbares Speichermedium gemäß Hauptanspruch und nebengeordneten Ansprüchen. Vorteilhafte Ausgestaltungen sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind gleichermaßen auf die anderen beanspruchten Gegenstände zu übertragen und umgekehrt. Mit anderen Worten können gegenständliche Ansprüche (die beispielsweise auf eine Sprachsteuervorrichtung gerichtet sind) auch mit Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Merkmale, bspw. Module oder Einheiten, einer der Vorrichtungen ausgebildet.

Die vorliegende Erfindung betrifft in einem ersten Aspekt ein Verfahren zur Sprachsteuerung einer medizinischen Vorrichtung. Das Verfahren ist in Ausführungen als computerimplementiertes Verfahren ausgebildet. Das Verfahren umfasst eine Vielzahl von Schritten.

Ein Schritt ist auf ein Erfassen eines Audiosignals enthaltend eine auf die Steuerung der Vorrichtung gerichtete Spracheingabe einer Bedienperson. Ein Schritt ist auf ein Analysieren des Audiosignals zum Bereitstellen eines ersten Sprachanalyseergebnisses gerichtet. Ein Schritt ist auf ein Erkennen eines ersten Sprachbefehls basierend auf dem ersten Sprachanalyseergebnis gerichtet. Ein Schritt ist auf ein Zuordnen des ersten Sprachbefehls zu einer Sicherheitsklasse zu dem ersten Sprachsteuerbefehl gerichtet, wobei wenigstens eine Sicherheitsklasse für sicherheitskritische Sprachbefehle vorgesehen ist. Ein Schritt ist auf ein Ermitteln eines Verifikationssignals zur Bestätigung des ersten Sprachbefehls gerichtet.

Dieser Schritt wird ausgeführt, wenn der erste Sprachbefehl der Sicherheitsklasse für sicherheitskritische Sprachbefehle zugeordnet wurde. Sicherheitskritische Sprachbefehle umfassen in Ausführungen der Erfindung Sprachbefehle für die medizinische Vorrichtung, die insbesondere erstfehlersicher ausgebildet werden müssen.

Ein Schritt ist auf ein Erzeugen eines Steuersignals zur Steuerung der medizinischen Vorrichtung basierend auf dem ersten Sprachbefehl und dem Verifikationssignal gerichtet.

Dieser Schritt wird ausgeführt, sofern der erste Sprachbefehl anhand des Verifikationssignals bestätigt wurde. Dabei ist das Steuersignal geeignet bzw. ausgebildet, die medizinische Vorrichtung entsprechend dem ersten Sprachbefehl zu steuern. Ein weiterer Schritt ist auf eine Eingabe des Steuersignals in die medizinische Vorrichtung gerichtet.

Ein Audiosignal im Sinne der Erfindung kann insbesondere eine Schallinformation enthalten. Das Audiosignal kann ein analoges oder digitales bzw. digitalisiertes Signal sein. Das digitalisierte Signal kann ausgehend von dem analogen Signal z.B. durch einen Analog-Digital-Wandler erzeugt werden. Entsprechend kann der Schritt des Erfassens ein Bereitstellen eines digitalisierten Audiosignals basierend auf dem empfangenen Audiosignal bzw. ein Digitalisieren des empfangenen Audiosignals umfassen. Das Erfassen des Audiosignals kann in Ausführungen ein Registrieren des Audiosignals mittels eines geeigneten Sensors, bspw. eines akustischen Sensors in Form eines Mikrophons umfassen, welches Bestandteil einer Benutzerschnittstelle der medizinischen Vorrichtung sein kann. Das Erfassen des Audiosignals kann ferner ein Bereitstellen des digitalen oder digitalisierten Signals für weitere Analyseschritte umfassen.

Das Audiosignal kann eine Kommunikation von einem Nutzer (im Folgenden auch Bedienperson), bspw. eine auszuführende Anweisung oder Angaben bezüglich der auszuführenden Anweisung, bspw. eine Sprachsteuerinformation, umfassen. Mit anderen Worten kann das Audiosignal eine Spracheingabe der Bedienperson in natürlicher Sprache umfassen. Als natürliche Sprache wird typischerweise eine von Menschen gesprochene Sprache bezeichnet. Natürliche Sprache kann einen Tonfall und/oder eine Sprachmelodie zur Modulation der Kommunikation aufweisen. Natürliche Sprachen können im Gegensatz zu formalen Sprachen strukturelle und lexikalische Uneindeutigkeiten aufweisen.

Ein Analysieren eines Audiosignals ist im Sinne der Erfindung auf das Erschließen bzw. das Erkennen eines Inhaltes des Spracheingabe der Bedienperson gerichtet. Das Analysieren kann das Erkennen einer menschlichen Stimme umfassen. Das Analysieren kann ein Analysieren von Werten der Spracheingabe wie bspw. Frequenz, Amplitude, Modulation oder dergleichen umfassen, die charakteristisch für die menschliche Sprache sind. Das Ergebnis des Analysierens wird zumindest teilweise in Form eines Sprachanalyseergebnisses bereitgestellt.

Das erfindungsgemäße Analysieren kann ein Verfahren zur Verarbeitung natürlicher Sprache anwenden. Insbesondere kann gemäß dem ersten Aspekt der Erfindung ein erster Computerlinguistik-Algorithmus auf das Audiosignal bzw. die Spracheingabe angewandt werden. Eine Möglichkeit zur Verarbeitung der in natürlicher Sprache formulierten Spracheingabe zur Bereitstellung des Sprachanalyseergebnisses ist ein Umwandeln der in natürlicher Sprache formulierten Spracheingabe in Text, also in strukturierte Sprache, mittels eines Sprache-zu-Text (Software-)Moduls. Anschließend kann eine weitere Analyse zur Bereitstellung des Sprachanalyseergebnisses, beispielsweise mittels latenter semantischer Analyse (engl. latent semantic indexing, kurz LSI), dem Text eine Bedeutung beimessen. Insbesondere wird hierbei das Audiosignal als Ganzes analysiert. Es kann vorgesehen sein, dabei einzelne Worte oder Wortgruppen und/oder eine Syntax zu erkennen. Die Beziehung/Stellung von Worten oder Wortgruppen zu vorher oder später in dem Audiosignal erscheinenden Worten oder Wortgruppen kann ebenfalls berücksichtigt werden. Die Analyse des Audiosignals kann insbesondere eine grammatikalische Analyse bspw. unter Anwendung eines Sprach- oder Grammatikmodells umfassen. Zusätzlich oder alternativ kann eine Sprachmelodie der Bedienperson ausgewertet werden. Derart realisiert die Erfindung ein Verstehen der in der Spracheingabe enthaltenen natürlichen Sprache (engl.: natural language understanding, NLU).

Der Schritt des Erkennens eines ersten Sprachbefehls basierend auf dem ersten Sprachanalyseergebnis ist darauf gerichtet, anhand des Sprachanalyseergebnisses der in der Spracheingabe erkannten Bedeutung des umfassten Textes einen eindeutigen Sprachbefehl zuzuordnen bzw. mit einem vordefinierten Satz möglicher Sprachbefehle in Verbindung zu bringen. Dabei kann der Spracheingabe im Schritt des Erkennens des ersten Sprachbefehls basierend auf dem Sprachanalyseergebnis eine beliebige Vielzahl von verschiedenen Sprachbefehlen zugeordnet werden. Jeder Sprachbefehl ist dabei repräsentativ für eine auszuführende Anweisung zur Ausführung eines Arbeitsschritts, einer definierten Aktion, Operation oder Bewegung, die basierend auf dem Sprachbefehl automatisch von der medizinischen Vorrichtung ausgeführt wird. Das Erkennen des ersten Sprachbefehls kann eine Klassifizierung der Spracheingabe anhand des Sprachanalyseergebnisses in eine Befehlsklasse eines vordefinierten Satzes verschiedener Befehlsklassen umfassen. Der vordefinierte Satz möglicher Befehlsklassen kann z.B. in Form einer Befehlsbibliothek vorliegen. Insbesondere können mehrere voneinander abweichende Spracheingaben bzw. ihre individuellen Sprachanalyseergebnisse ihrem Bedeutungsgehalt nach derselben Befehlsklasse und damit demselben Sprachbefehl zugeordnet werden.

Derart wird der Dimensionalität der natürlichen Sprache Rechnung getragen, bei der unter anderem mittels unterschiedlicher Wortwahl oder Sprachmelodie ein- und dieselbe Nutzerintention ausgedrückt werden kann.

Die Befehlsbibliothek kann auch eine Befehlsklasse für nicht erkannte Sprachbefehle umfassen, die immer dann zugeordnet wird, wenn das Audiosignal akustische Merkmale aufweist, die unspezifisch für einen konkreten Sprachbefehl sind bzw. sich nicht einer anderen Befehlsklasse zuordnen lässt.

Auch der Schritt des Erkennens des ersten Sprachbefehls kann durch Anwenden des ersten Computerlinguistik-Algorithmus ausgeführt werden. Der Schritt des Erkennens des ersten Sprachbefehls folgt dabei bevorzugt dem Erstellen eines Transkripts (strukturierte Sprache) des Sprachsignals bzw. dem auf dem Transkript basierenden Erstellen eines semantischen Analyseergebnisses. Beides kann in dem ersten Sprachanalyseergebnis umfasst sein.

Die Vielzahl der Befehlsklassen ist in Implementierungen zumindest teilweise spezifisch für die medizinische Vorrichtung und richtet sich insbesondere an der bereitgestellten Funktionalität der medizinischen Vorrichtung aus. Die Vielzahl der Befehlsklassen ist in Implementierungen anpassbar ausgebildet und kann insbesondere über die Auswahl bzw. die Konfiguration bzw. das Training des bei der Analyse eingesetzten Computer-linguistik-Algorithmus angepasst werden. Bspw. können weitere Befehlsklassen hinzugefügt werden.

Erfindungsgemäß ist jede Befehlsklasse bzw. jeder Sprachbefehl einer Sicherheitsklasse zugeordnet. Eine Sicherheitsklasse steht jeweils für ein Sicherheitsmaß bzw. Sicherheitslevel, welches bei Ausführung des/der dem jeweiligen Sprachbefehl entsprechenden Arbeitsschritt, Operation bzw. Aktion durch die medizinische Vorrichtung eingehalten werden muss. Das jeweilige Sicherheitsmaß kann dabei ein mittels Normierung festgelegtes Sicherheitsmaß sein. Erfindungsgemäß sind mindestens zwei Sicherheitsklassen vorgesehen. Die zwei Sicherheitsklassen teilen die Sprachbefehle bzw. ihre Befehlsklassen in sicherheitskritische umfassend erstfehlersichere Sprachbefehle und nicht sicherheitskritische Sprachbefehle ein. Vorteilhaft sind mehr als zwei Sicherheitsklassen jeweils entsprechend verschiedener Sicherheitslevel bzw. Sicherheitsstufen vorgesehen. Auch hier ist mindestens eine Sicherklasse für sicherheitskritische umfassend erstfehlersichere Sprachbefehle vorgesehen, wobei weitere Befehlsklassen für sicherheitskritische Sprachbefehle vorgesehen sein können, wobei die Sicherheitsanforderungen für die Gewährleistung der Sicherheitsanforderungen in Abhängigkeit einer Befehlsklasse unterschiedlich hoch sein können. Die Sicherheitsanforderungen sind für die Befehlsklasse umfassend die erstfehlersicheren Sprachbefehle am höchsten.

Die Zuordnung einer Befehlsklasse zu einer Sicherheitsklasse erfolgt in Implementierungen basierend auf einer vorab festgelegten Zuordnungsvorschrift, die die Art des Sprachbefehls bzw. ein Gefährdungsmaß der durch den Sprachbefehl auszulösenden Operation bzw. Aktion bzw. Bewegung der medizinischen Vorrichtung für den Patienten und/oder das Bedienpersonal und/oder die medizinische Vorrichtung oder für anderes medizinisches Equipment berücksichtigt. In Implementierungen erfolgt die Zuordnung über eine vorab festgelegte Lookup-Tabelle. In weiteren Implementierungen können vorab definierte Schlüsselwörter vorgesehen sein, die von dem ersten Computer-linguistik-Algorithmus in dem Audiosignal erkannt werden können, die jeweils mit einer der Sicherheitsklassen verknüpft sind. Einer Sicherheitsklasse können dabei ein oder mehrere Schlüsselwörter zugeordnet sein. Dem ersten Sprachbefehl wird in Ausführungen also eine Sicherheitsklasse zugeordnet, wenn ein oder mehrere Schlüsselwörter durch den Computer-Linguistik-Algorithmus in dem Audiosignal erkannt wurden. Alternativ kann die Sicherheitsklasse bei Erkennung des ersten Sprachbefehls automatisch per vorab definierter Zuordnungsvorschrift festgelegt sein.

Der Schritt des Zuordnens des ersten Sprachbefehls zu einer Sicherheitsklasse ist folglich darauf gerichtet, den ersten Sprachbefehl als sicherheitskritischen, insbesondere als erstfehlersicheren, oder nicht sicherheitskritischen Sprachbefehl zu identifizieren. In Implementierungen wird auch dieser Schritt durch den ersten Computerlinguistik-Algorithmus ausgeführt.

Wird der erste Sprachbefehl als sicherheitskritischer Sprachbefehl erkannt, wird in einem weiteren Schritt ein Verifikationssignal zur Bestätigung des ersten Sprachbefehls ermittelt. In diesem Schritt wird überprüft, ob der erste, identifizierte Sprachbefehl tatsächlich der von der Bedienperson gewünschten Nutzerintention bzw. dem gewünschten Kommando entspricht. Das Verifikationssignal gibt dabei ein Maß für die Übereinstimmung zwischen gewünschter Benutzerintention und dem ersten Sprachbefehl an. In Implementierungen kann das Verifikationssignal eine Verifikationsinformation umfassen. Die Verifikationsinformation kann wenigstens zwei, bspw. ‚1' für vollständige Übereinstimmung und '0' für keine Übereinstimmung, in Ausführungen auch mehrere verschiedene diskrete Werte, bspw. zwischen '1' und '0' annehmen. Einige der diskreten Werte können verschiedenen Übereinstimmungsniveaus entsprechen, die jeweils einer anteiligen, nicht vollständigen Übereinstimmung entsprechen können. Auch diese Werte können in Ausführungen der Erfindung einer auseichenden Übereinstimmung zwischen Sprachbefehl und gewünschter Nutzerintention entsprechen, sofern dem jeweiligen Sprachbefehl ein entsprechend niedriges Sicherheitslevel entsprechend einer der möglichen Sicherheitsklassen zugeordnet ist.

Erfindungsgemäß wird das Verifikationssignal zur Weiterverarbeitung bspw. einer Steuereinheit einer erfindungsgemäßen Sprachsteuervorrichtung bereitgestellt. Insofern kann der Schritt des Erzeugens eines Steuersignals basierend auf dem Verifikationssignal und dem ersten Sprachbefehl ein Bereitstellen derselben umfassen. Das Verifikationssignal zeigt an, ob das für den ersten Sprachbefehl geforderte Übereinstimmungsmaß erreicht ist oder nicht.

Bei entsprechender Bestätigung des ersten Sprachbefehls mittels des Verifikationssignals, wird in einem folgenden Schritt ein Steuersignal zur Steuerung der medizinischen Vorrichtung basierend auf dem ersten Sprachbefehl und dem Verifikationssignal erzeugt und in die medizinische Vorrichtung eingegeben.

Mit dem Schritt des Ermittelns des Verifikationssignals wendet die vorliegende Erfindung vorteilhaft eine Überwachung bzw. Plausibilisierung eines Sprachbefehls, also eines mittels Methoden der maschinellen Sprachanalyse gewonnenen Steuerbefehls an. Mit anderen Worten ermöglicht die vorliegende Erfindung mit diesen Schritten, einen Prüf- (P-protect) Pfad für Sprachbefehle entsprechend eines erstfehlersicheren Systems zu implementieren. Wird der erste Sprachbefehl durch den Plausibilisierungsschritt bestätigt (Verifikationssignal zeigt eine Übereinstimmung), wird der erste Sprachbefehl weiterverarbeitet und durch die medizinische Vorrichtung ausgeführt. Wird der erste Sprachbefehl durch den Plausibilisierungsschritt nicht bestätigt (Verifikationssignal zeigt eine Abweichung), wird der Prozess abgebrochen und der erste Sprachbefehl wird verworfen und nicht ausgeführt.

Die Erfindung ermöglicht mit der Ermittlung des Verifikationssignals vorteilhaft, dass die Ausführung von mittels maschineller Spracherkennung falsch erkannten bzw. falsch interpretierten Nutzereingaben verhindert wird. Insbesondere wird derart die sicherheitskritische bzw. erstfehlersichere Ausführung von Steuerbefehlen gewährleistet. Der erste erkannte Sprachbefehl wird nur dann ausgeführt, wenn dieser mittels des Plausibilisierungsschritts bestätigt wurde.

Das Ermitteln des Verifikationssignals wird in Ausführungen der Erfindung in einem getrennten, unabhängigen Hard- und/oder Softwaresystem implementiert. Insbesondere die Ermittlung des Verifikationssignals erfolgt also unabhängig von den vorherigen Prozessschritten. Algorithmen zur Ermittlung des Verifikationssignals unterscheiden sich insbesondere von den zuvor im Verfahren eingesetzten Computerlinguistik-Algorithmen. Der Schritt des Ermittelns des Verifikationssignals wird in Ausführungen der Erfindung in einer anderen realen oder virtuellen Recheneinheit ausgeführt als die vorherigen Prozessschritte. Dadurch kann erfindungsgemäß ein erstfehlersicheres System umfassend einen Steuer-Pfad (C-Pfad, C-control) und einen Prüf-Pfad (P-Pfad, P-protect) eingerichtet werden, wobei der Schritt des Ermittelns des Verifikationssignals innerhalb des P-Pfades erfolgt. Insbesondere die Schritte des Erfassens eines Audiosignals enthaltend eine auf die Steuerung der Vorrichtung gerichtete Spracheingabe einer Bedienperson, des ersten Analysierens des Audiosignals zum Bereitstellen eines ersten Sprachanalyseergebnisses, des Erkennens eines ersten Sprachbefehls basierend auf dem ersten Sprachanalyseergebnis und des Zuordnens des ersten Sprachbefehls zu einer Sicherheitsklasse, wobei eine Sicherheitsklasse insbesondere für erstfehlersichere Sprachbefehle vorgesehen ist, bilden einen wesentlichen Bestandteil des C-Pfades.

Der Schritt des Ermittelns des Verifikationssignals kann erfindungsgemäß auf verschiedene Weise ausgeführt werden, wie im Folgenden ausführlicher beschrieben wird.

Zum einen kann das Ermitteln des Verifikationssignals das Erfassen und Auswerten einer weiteren Nutzereingabe umfassen. In dieser Ausführung wird die Bestätigung des ersten Sprachbefehls basierend auf dieser Nutzereingabe ermittelt. Alternativ oder zusätzlich kann das Ermitteln eine weitere, von der ersten Analyse des Audiosignals unabhängige zweite maschinelle Sprachanalyse umfassen, wobei ein zweiter Sprachbefehl abgeleitet wird. Hierbei wird die Bestätigung des ersten Sprachbefehls basierend auf einem Vergleich des ersten und des zweiten Sprachbefehls abgeleitet.

Erfindungsgemäß umfasst das Ermitteln des Verifikationssignals, den ersten Sprachbefehl und eine Aufforderung zur Bestätigung des ersten Sprachbefehls an die Bedienperson auszugeben. Die Ausgabe des ersten Sprachbefehls an die Bedienperson dient dazu, der Bedienperson zur Kenntnis zu bringen, welcher Sprachbefehl basierend auf der analysierten Spracheingabe automatisch maschinell als erster Sprachbefehl erkannt wurde.

Daneben umfasst die Ausgabe eine Aufforderung zur Bestätigung des ersten Sprachbefehls per Benutzereingabe. Mit anderen Worten wird in Implementierungen basierend auf dem ersten erkannten Sprachbefehl wenigstens ein entsprechendes Steuersignal für eine insbesondere akustische Ausgabevorrichtung einer Benutzerschnittstelle einer erfindungsgemäßen Sprachsteuervorrichtung oder einer medizinischen Vorrichtung erzeugt, basierend auf welchem die Ausgabeeinheit Ausgabedaten erzeugt.

Erfindungsgemäß umfasst das Ermitteln des Verifikationssignals auch ein Erfassen einer Benutzereingabe. Diese ist auf die Bestätigung des ersten Sprachbefehls durch die Bedienperson gerichtet. Wird der ausgegebene erste Sprachbefehl durch die Bedienperson als der mit dem gewünschten Kommando übereinstimmende Sprachbefehl erkannt, kann die Bedienperson eine der ausgegebenen Aufforderung entsprechende Bestätigung erteilen, die über eine Eingabeeinheit der Benutzerschnitte der medizinischen Vorrichtung als Benutzereingabe registriert und weiterverarbeitet werden kann, insbesondere mittels einer Auswerteeinheit (oder eines Submoduls derselben) der Sprachsteuervorrichtung. Besonders bevorzugt umfasst in Ausführungen der Erfindung das Ausgeben ein Ausgeben eines auf dem ersten Sprachbefehl basierenden Audiosignals, und/oder umfasst das Erfassen ein Erfassen einer als Audiosignal ausgebildeten Benutzereingabe. Mit anderen Worten wird in Ausführungen auch der Plausibilisierungsschritt des erfindungsgemäßen Verfahrens mit Mitteln bzw. Algorithmen der maschinellen Sprachverarbeitung ausgeführt, bei dem in einem Teilschritt ein Audiosignal in Form natürlicher Sprache generiert wird.

Gemäß einiger Implementierungen der Erfindung wird also basierend auf dem ersten erkannten Sprachbefehl ein Algorithmus zur Sprachgenerierung angewandt, welcher ein Audiosignal umfassend natürliche Sprache erzeugt (Natural Language Generation, NLG). Es erfolgt also eine Umwandlung von Text-zu-Sprache. Die Generierung von Sprache aus einem (strukturierten) Text kann in Ausführungen mittels eines weiteren, zu einem ersten Computer-Linguistik-Algorithmus invers agierenden, Computerlinguistik-Algorithmus ausgeführt werden. Das Audiosignal kann in Ausführungen ein Ausgeben des Audiosignals mittels eines geeigneten Schallwandlers, bspw. eines Lautsprechers umfassen, welcher ebenfalls Bestandteil einer Benutzerschnittstelle sein kann.

In einigen Implementierungen der Erfindung wird auch die Benutzereingabe als Spracheingabe getätigt und ein entsprechendes Audiosignal erfasst. Die Benutzereingabe kann also in Form eines Audiosignals erfolgen und umfasst natürliche Sprache, wie weiter oben beschrieben, und einen Tonfall und/oder eine Sprachmelodie zur Modulation der Kommunikation. Die Benutzereingabe kann also auch eine Schallinformation enthalten und ein analoges oder digitales bzw. digitalisiertes Signal sein. Das digitalisierte Signal kann ausgehend von dem analogen Signal z.B. durch einen Analog-Digital-Wandler erzeugt werden und das Erfassen kann ein Bereitstellen eines digitalisierten Audiosignals basierend auf dem empfangenen Audiosignal bzw. ein Digitalisieren des empfangenen Audiosignals umfassen. Das Erfassen der Benutzereingabe kann in Ausführungen mittels des eingangs beschriebenen Mikrophons der Benutzerschnittstelle erfolgen.

Die erfasste Benutzereingabe wird anschließend analysiert, um seinen Inhalt zu erschließen. Das Analysieren kann auch hier, wie eingangs beschrieben, ein Analysieren von Werten der Spracheingabe wie bspw. Frequenz, Amplitude, Modulation oder dergleichen umfassen, die charakteristisch für die menschliche Sprache sind. Das Analysieren kann ebenfalls mittels eines weiteren Computerlinguistik-Algorithmus erfolgen, der wie weiter oben bereits beschrieben ausgebildet sein kann. Dieser weitere Computerlinguistik-Algorithmus kann in Ausführungen ausgebildet sein, insbesondere kurze, auf eine Befehlsbestätigung gerichtete Schlüsselworte wie 'Ja', 'Yes', 'Confirmed', 'Check' oder dergleichen zu erkennen.

In alternativen Implementierungen der Erfindung kann sowohl die Ausgabe des ersten Sprachbefehls als auch das Erfassen der bestätigenden Benutzereingabe andersartig ausgebildet sein. Bspw. kann eine Ausgabe des ersten Sprachbefehls alternativ mittels eines graphischen Displays erfolgen, welches ebenfalls Bestandteil der Benutzerschnittstelle sein kann. Diese Ausführung umfasst eine entsprechende Umwandlung des ersten Sprachbefehls in Steuersignale bzw. graphische Ausgabedaten für das Display. In weiteren Implementierungen kann das Erfassen der Benutzereingabe ein maschinelles Erkennen einer Geste der Bedienperson umfassen oder das Registrieren eines Knopfdrucks oder einer Berührung eines als Touchdisplay ausgebildetes Display umfassen. Entsprechend umfasst ein Erfassen der Benutzereingabe ein jeweiliges Erkennen oder Registrieren der Benutzereingabe mittels eines optischen Sensors (wie bspw. einer Kamera), eines resistiven oder kapazitiven Sensors oder eines Druck- oder Kraftsensors oder dergleichen und eine Umwandlung des jeweils erfassten Signals in ein weiterverarbeitbares elektrisches Signal.

Weitere Ausbildungsvarianten sind möglich, bei denen insbesondere jeweils eine akustische Ausgabe des ersten Sprachbefehls oder eine akustische Erfassung der auf die Bestätigung gerichteten Benutzereingabe jeweils mit andersartig ausgebildeten Ausgabe- bzw. Erfassungsvarianten kombinierbar sind.

Erfindungsgemäß bestätigt das Verifikationssignal den ersten, erkannten Sprachbefehl dann, wenn die Benutzereingabe der Bedienperson

- ein vorab definiertes Zeitkriterium erfüllt und, in bevorzugten Implementierungen, zusätzlich

- ein vorab definiertes Inhaltskriterium erfüllt.

Mit anderen Worten durchläuft das erfindungsgemäße Verfahren einen Prüfschritt, in dem die Benutzereingabe wenigstens eine zeitliche Erwartung und eventuell zusätzlich eine inhaltliche Erwartung erfüllen muss. Das inhaltliche und/oder das zeitliche Kriterium sind in bevorzugten Implementierungen abhängig von dem ersten erkannten Sprachbefehl bzw. der ihm zugewiesenen Sicherheitsklasse. Bei einem Steuerbefehl, von dem bei unbeabsichtigter Ausführung durch die medizinische Vorrichtung ein hohes Risiko insbesondere für Patient oder Bedienperson ausgeht, wird die auf die Bestätigung gerichtete Benutzereingabe erfindungsgemäß ein strengeres Zeitkriterium erfüllen müssen als ein Steuerbefehl, durch den bei unbeabsichtigter Ausführung ein geringeres Risiko ausgeht.

Ein Zeitkriterium kann in Ausführungen durch einen vorab entsprechend einem jeweiligen Sprachbefehl bzw. einer Befehlsklasse festgelegten zeitlichen Schwellwert bzw. einer Zeitspanne entsprechen, innerhalb welcher die Benutzereingabe erfolgen muss. Einem Sprachbefehl einer höheren Sicherheitsstufe/Sicherheitsklasse ist bspw. ein geringerer zeitlicher Schwellwert zugeordnet als einem Sprachbefehl einer niedrigen Sicherheitsstufe. Dieses Vorgehen basiert auf der Überlegung, dass eine Bestätigung eines korrekt erkannten und mit der gewünschten Nutzerintention übereinstimmenden Sprachbefehls quasi instantan bzw. sehr schnell erfolgen kann und eine Verzögerung der Bestätigung in Bereichen oberhalb des zeitlichen Schwellwertes typischerweise zumindest Unsicherheit in Bezug auf den ersten Sprachbefehl bei der Bedienperson bzw. sogar eine Diskrepanz zwischen gewünschter Nutzerintention und erstem, erkannten Sprachbefehl anzeigt. Erfolgt die Benutzereingabe also nicht innerhalb der vorgegebenen Zeitspanne oder nach einer als Abbruchkriterium definierten Zeitspanne, wird der erste Sprachbefehl verworfen und nicht ausgeführt.

In einigen Implementierungen kann die Erfindung die Ausgabe der Aufforderung zur Bestätigung des ersten Sprachbefehls auch die entsprechende Zeitspanne gemäß den Zeitkriterium umfassen, in der die Benutzereingabe erfolgen muss.

Ein Inhaltskriterium ist in bevorzugten Ausführungen der Erfindung vorgesehen, bei denen die auf die Bestätigung gerichtete Benutzereingabe in Form eines Audiosignals getätigt wird bzw. eine Inhaltsanalyse in Bezug auf die Benutzereingabe erfolgt. Ein Inhaltskriterium kann insbesondere in Form eines oder mehrerer von dem jeweiligen Steuerbefehl bzw. seiner Sicherheitsklasse abhängigen und vorab definierten Schlüsselworten bzw. Wortfolgen vorliegen. Werden diese mittels des weiteren Computerlinguistik-Algorithmus, der auf die Benutzereingabe angewandt wird, erkannt, ist das Inhaltskriterium erfüllt. Werden die Schlüsselworte nicht erkannt, wird der erste Sprachbefehl verworfen und nicht durch die medizinische Vorrichtung ausgeführt.

Ein Inhaltskriterium kann in Ausführungen der Erfindung bspw. auch darin bestehen, dass ein zuvor definierter Druck- oder Kraftschwellwert an einem entsprechenden Sensor der Bedienschnittstelle erreicht bzw. überschritten wird. Entsprechend kann es in bevorzugten Implementierungen der Erfindung vorgesehen sein, dass die Ausgabe der Aufforderung zur Bestätigung des ersten Sprachbefehls auch eine Angabe umfasst, wie, bspw. mit welchen Schlüsselworten oder mit welchem Druck die bestätigende Benutzereingabe erfolgen muss.

Insbesondere für Sprachbefehle mit hoher Sicherheitsstufe kann für eine Validierung des ersten Sprachbefehls die Erfüllung eines Inhaltskriteriums und eines Zeitkriteriums erforderlich sein, damit der erste Sprachbefehl ausgeführt werden kann. Insbesondere kann in Ausführungen der Erfindung für das Zeitkriterium eine Zeitspanne derart gewählt werden, dass sie eine zu erwartende Länge einer Benutzereingabe in Form einer Spracheingabe berücksichtigt.

Im Anschluss an die Prüfung des Inhalts- und/oder Zeitkriteriums, wird das Verifikationssignal erzeugt, das in Abhängigkeit des Prüfergebnisses eine Verifikationsinformation umfasst, die bspw. einen vorab definierten diskreten Wert, bspw. ‚1' bei vollständiger Übereinstimmung zwischen gewünschter Nutzerintention und erstem Sprachbefehl, einnimmt.

Der Schritt des Ermittelns eines Verifikationssignals wird in Ausführungen der Erfindung klassisch implementiert. Eine Überwachung des Inhalts- und/oder des Zeitkriteriums ist daher mit konventionellen Methoden nachweisbar und sicherheitstechnisch belastbar, obwohl auch das Verifikationssignal auf einem Steuerbefehl in Form des ersten Sprachbefehls basiert, der mittels einer maschinellen Sprachanalyse, also mittels einer sicherheitstechnisch nicht belastbaren Software-Komponente, erzeugt wurde. Die Belastbarkeit wird insbesondere durch die vorab festgelegten zeitlichen und inhaltlichen Sicherheitskriterien gewährleistet. Derart reduziert die vorliegende Erfindung die Wahrscheinlichkeit, dass ein unrichtig erkannter Sprachbefehl durch die medizinische Vorrichtung tatsächlich ausgeführt wird.

Erfindungsgemäß ist das Zeitkriterium in Abhängigkeit des ersten Sprachbefehls anpassbar ausgebildet. Das erfindungsgemäße Verfahren kann in Ausführungen folglich einen Anpassschritt umfassen, in welchem das Zeitkriterium basierend auf bspw. Benutzerspezifischen Vorgaben oder einer Nutzereingabe angepasst bzw. verändert werden kann. Erfindungsgemäß ist das Zeitkriterium in Abhängigkeit des ersten Sprachbefehls nach Benutzer-spezifischen Vorgaben anpassbar.

Mit anderen Worten kann ein zeitlicher Schwellwert, der für die Validierung des ersten Sprachbefehls vorab, bspw. basierend auf Erfahrungswerten festgelegt wurde, (nachträglich) angepasst, insbesondere vergrößert, werden. Damit wird der Bedienperson größere Flexibilität bei der Erteilung der Bestätigung gegeben. Die Bedienperson hat dadurch mehr Zeit, den ersten Sprachbefehl zu bestätigen. Dieses Vorgehen bewirkt eine höhere Nutzerfreundlichkeit und damit Akzeptanz der technischen Lösung im medizinischen Alltag, insbesondere dann, wenn die Bedienperson für die gesamte medizinische Workflow- und Patientenüberwachung verantwortlich ist. Die Anpassung des zeitlichen Kriteriums kann insbesondere bei Sprachbefehlen vorgesehen sein, die einer niedrigeren Sicherheitsstufe zugeordnet sind. Alternativ oder zusätzlich kann für jede der Befehlsklassen ein Wertebereich für eine zulässige Zeitspanne des Zeitkriteriums hinterlegt sein. Eine Anpassung des Zeitkriteriums kann in diesen Ausführungen nur innerhalb des Wertebereichs erfolgen.

In weiteren Ausführungen kann für bestimmte Sprachbefehle, insbesondere Sprachbefehle einer hohen Sicherheitsstufe, vorgesehen sein, dass die Bedienperson mehrmals eine bestätigende Benutzereingabe vornehmen muss, wobei jede der Benutzereingaben mit einem inhaltlichen und/oder zeitlichen Kriterium belegt sein kann, welches jeweils im Sinne einer Bestätigung des ersten Sprachbefehls erfüllt werden muss. Entsprechend kann das Ermitteln des Verifikationssignals mehrere, bspw. zwei oder drei Bestätigungszyklen umfassen.

Entsprechend kann das Ermitteln des Verifikationssignals ein mehrfaches Ausgeben einer wie oben beschrieben ausgebildeten Aufforderung zur Bestätigung des ersten Sprachbefehls an die Bedienperson umfassen, also eine Aufforderung pro Bestätigungszyklus. Alternativ oder zusätzlich kann das Ermitteln des Verifikationssignals ein mehrfaches Erfassen einer bestätigenden Benutzereingabe mit den wie oben beschriebenen Mitteln umfassen, also ein Erfassen pro Bestätigungszyklus.

Auch die Anzahl der Bestätigungszyklen kann in Ausführungen der Erfindung unter Berücksichtigung des jeweiligen Sprachbefehls bzw. seiner Sicherheitsstufe anpassbar ausgebildet sein.

Derart ist das erfindungsgemäße Verfahren skalierbar und kann entsprechend einer Nutzerpräferenz im Hinblick auf eine höhere Bedienfreundlichkeit (weniger Bestätigungszyklen und/oder größere Zeitschwellwerte) und/oder auf eine höhere Sicherheit (mehr Bestätigungszyklen und/oder kleinere Zeitschwellwerte) modifiziert werden. Damit kann auch die Skalierbarkeit der erfindungsgemäßen Lehre zu einer höheren sicherheitstechnischen Belastbarkeit der Validierung des ersten Sprachbefehls entsprechend einem klassischen P-Pfad beitragen.

Weitere Implementierungen der Erfindung sehen vor, dass das Ermitteln des Verifikationssignals auch umfasst, eine Aufforderung zur Benutzereingabe einer für den erkannten Sprachbefehl spezifischen Sprachsteuerinformation an die Bedienperson auszugeben, und eine die Sprachsteuerinformation umfassende Benutzereingabe zu erfassen. Die Erfassung von spezifischer Sprachsteuerinformation ist besonders gut für eine Steuerung der medizinischen Vorrichtung mittels Sprache geeignet, denn die Bedienperson benötigt dazu keine Hand. Diese kann vorteilhaft bspw. am Patienten verbleiben. Daneben kann spezifische Sprachsteuerinformation zeitgleich bzw. direkt zusammen mit der Bestätigung der ersten Sprachbefehls eingegeben werden, was die Bedienung insgesamt erleichtert.

Die Ausgabe der Aufforderung zur Benutzereingabe der Sprachsteuerinformation ist bevorzugt von dem ersten Sprachbefehl abhängig. Für einen Sprachsteuerbefehl bzw. jede Befehlsklasse kann bspw. ein vorab definierter Datenstrom repräsentierend die Aufforderung zur Eingabe von Sprachsteuerinformation hinterlegt sein, der jeweils mittels des oben beschriebenen Algorithmus zur Sprachgenerierung (NLG), aus dem im Datenstrom enthaltenen Text ein Audiosignal umfassend natürliche Sprache erzeugt wird. Eine Sprachsteuerinformation ist eine für die Umsetzung des ersten Sprachbefehls erforderliche Angabe bzw. Informationen. Die Sprachsteuerinformation kann bspw. die Einstellung eines oder mehrerer Betriebsparameter der medizinischen Vorrichtung betreffen, bspw. kann die Sprachsteuerinformation die Länge eines Verfahrweges für eine Verstellbewegung der medizinischen Vorrichtung angeben oder dergleichen. Entsprechend kann der weitere Computerlinguistik-Algorithmus ausgebildet sein, der die Sprachsteuerinformation umfassende Benutzereingabe zu analysieren und die Sprachsteuerinformation zu identifizieren.

Dazu kann der weitere Computerlinguistik-Algorithmus wieder ausgebildet sein, Schlüsselworte oder Zahlen zu erkennen, bspw. ‚5 mm' oder dergleichen.

In weiteren bevorzugten Implementierungen der Erfindung umfasst das Analysieren des Audiosignals ein Anwenden eines ersten Computerlinguistik-Algorithmus umfassend eine erste trainierte Funktion auf das Audiosignal, also einen trainierten Algorithmus des maschinellen Lernens. Bevorzugt umfassen auch das Erkennen eines ersten Sprachbefehls basierend auf dem ersten Sprachanalyseergebnis sowie das Zuordnen des ersten Sprachbefehls zu einer Sicherheitsklasse ein Anwenden des ersten Computerlinguistik-Algorithmus.

Bevorzugt umfasst die trainierte Funktion bzw. der trainierte Algorithmus des maschinellen Lernens ein neuronales Netz, bevorzugt ein faltendes neuronales Netz. Ein neuronales Netz ist i.W. wie ein biologisches neuronales Netz aufgebaut, bspw. wie das menschliche Gehirn. Bevorzugt umfasst ein künstliches, neuronales Netz eine Eingabeschicht und eine Ausgabeschicht. Dazwischen kann es eine Vielzahl von Zwischenschichten umfassen. Jede der Schichten umfasst einen, bevorzugt eine Vielzahl von Knoten. Jeder Knoten wird dabei als biologische Recheneinheit bzw. Schaltstelle, bspw. als Neuron angesehen. Anders ausgedrückt, entspricht ein einzelner Knoten einer bestimmten auf die Eingabedaten angewandten Rechenoperation. Knoten einer Schicht können untereinander über entsprechende Ränder oder Verbindungen miteinander und/oder zu Knoten anderer Schichten verbunden sein, speziell über gerichtete Verbindungen. Diese Ränder bzw. Verbindungen definieren den Datenfluß des Netzes. In bevorzugten Ausführungen ist ein Rand/eine Verbindung mit einem Parameter ausgestattet, wobei der Parameter auch als "Gewicht" bezeichnet wird. Dieser Parameter reguliert den Einfluss bzw. das Gewicht der Ausgabedaten eines ersten Knotens für die Eingabe eines zweiten Knotens, der über die Verbindung mit dem ersten Knoten in Kontakt steht.

Gemäß Ausführungen der Erfindung ist das neuronale Netz ein trainiertes Netz. Das Training des neuronalen Netzes wird bevorzugt im Sinne eines 'überwachten Lernens' basierend auf Trainingsdaten eines Trainingsdatensatzes, nämlich bekannten Paaren von Eingabe- und Ausgabedaten durchgeführt. Dabei werden die bekannten Eingabedaten als Eingabedaten an das neuronale Netz übergeben und die Ausgabedaten des neuronalen Netzes werden mit den bekannten Ausgabedaten des Trainingsdatensatzes verglichen. Das künstliche neuronale Netz lernt nun selbständig und passt die Gewichte der einzelnen Knoten bzw. Verbindungen derart und solange an, bis die Ausgabedaten der Ausgabeschicht des neuronalen Netzes ausreichend ähnlich zu den bekannten Ausgabedaten des Trainingsdatensatzes sind. In diesem Zusammenhang spricht man bei faltenden neuronalen Netzen auch von 'deep learning'. Die Termini 'neuronales Netz' und 'künstliches neuronales Netz' können als Synonyme verstanden werden.

Erfindungsgemäß wird in Ausführungen das faltende neuronale Netz, also die trainierte Funktion des ersten Computerlinguistik-Algorithmus in einer Trainingsphase darauf trainiert, das erfasste Audiosignal zu analysieren und einen ersten Sprachbefehl bzw. eine Befehlsklasse entsprechend dem ersten Sprachbefehl daraus zu erkennen. Der erste Sprachbefehl und/oder die Befehlsklasse entspricht dann den Ausgabedaten der trainierten Funktion. In anderen Ausführungen der Erfindung kann die trainierte Funktion zudem darauf trainiert werden, eine Sicherheitsklasse zu dem ersten Sprachbefehl zuzuordnen. Dann können die Ausgabedaten der trainierten Funktion des ersten Computerlinguistik-Algorithmus auch die Sicherheitsklasse umfassen. Sprachbefehle entsprechend der Gruppe von Steuerbefehlen, die der erste Computerlinguistik-Algorithmus erkennen soll, weisen können eine Vielzahl von Merkmalskombinationen aufweisen, also eine Vielzahl von verschiedenen Frequenzmustern, Amplituden, Modulationen, oder dergleichen. Entsprechend lernt die trainierte Funktion in der Trainingsphase, anhand einer aus dem Audiosignal extrahierten Merkmalskombination im Sinne des ersten Sprachanalyseergebnisses einen der möglichen Sprachbefehle zuzuordnen bzw. das Audiosignal entsprechend einer der Befehlsklassen zu klassifizieren bzw. das Audiosignal entsprechend einer Sicherheitsklasse zu klassifizieren. Die Trainingsphase kann auch eine manuelle Zuordnung von Trainings-Eingabedaten in Form von Spracheingaben zu einzelnen Sprachbefehlen bzw. Befehlsklassen umfassen.

Eine erste Gruppe der neuronalen Netz-Schichten kann auf das Extrahieren bzw. das Ermitteln der akustischen Merkmale eines Audiosignals gerichtet sein, also das Bereitstellen des Sprachanalyseergebnisses umfassend eine für das Audiosignal spezifische Kombination akustischer Merkmale. Das Sprachanalyseergebnis kann in Form eines akustischen Merkmalsvektors bereitgestellt werden. Insofern dient ein das Audiosignal bevorzugt vollständig umfassender Sprachdatenstrom als Eingabe-Daten für das neuronale Netz. Das Sprachanalyseergebnis kann als Eingabe-Daten für eine zweite Gruppe neuronaler Netz-Schichten dienen, die auch als ‚Classifier' bezeichnet werden. Die zweite Gruppe neuronaler Netz-Schichten dient dazu, dem extrahierten Merkmalsvektor wenigstens einen Sprachbefehl bzw. eine Befehlsklasse zuzuordnen. Die Menge von Befehlsklassen kann dabei insbesondere auch eine Befehlsklasse für nicht erkannte Sprachbefehle umfassen. Das neuronale Netz kann entsprechend darauf trainiert sein, das Audiosignal dieser Klasse zuzuordnen, wenn kein Sprachbefehl anhand der Merkmale eindeutig erkannt werden kann. Eine dritte Gruppe neuronaler Netz-Schichten kann ausgebildet sein, basierend auf der Befehlsklasse und/oder dem erkannten Sprachbefehl eine Sicherheitsklasse zuzuordnen, wobei insbesondere die ermittelte Befehlsklasse und/oder der erkannten Sprachbefehl als Eingabe-Daten für die dritte Gruppe neuronaler Netz-Schichten dienen. Die dritte Gruppe ist nun ausgebildet, die Befehlsklasse und/oder den erkannten Sprachbefehl als sicherheitskritischen, insbesondere erstfehlersicheren Befehl oder nicht sicherheitskritischen Befehl zu klassifizieren.

Die Analyseschritte bzw. die Funktionen können auch von mehreren, insbesondere zwei oder drei eigenständigen neuronalen Netzen ausgeführt werden. Der erste Computerlinguistik-Algorithmus kann folglich ein oder mehrere neuronale Netze umfassen. Zum Beispiel kann die Merkmalsextraktion mit einem ersten neuronalen Netz, und die Klassifizierung mit einem zweiten neuronalen Netz ausgeführt werden.

Das Klassifizieren eines Audiosignals in eine Befehlsklasse basierend auf dem ersten Sprachanalyseergebnis kann auf einem Vergleich des extrahierten Merkmalsvektors des Audiosignals mit in der Befehlsbibliothek hinterlegten, für jede Befehlsklasse spezifischen Merkmalsvektoren basieren. Für eine Befehlsklasse können ein oder mehrere Merkmalsvektoren hinterlegt sein, um der Vieldimensionalität der menschlichen Sprache Rechnung zu tragen und basierend auf einer Vielzahl von verschiedenartigen Spracheingaben einen konkreten Sprachbefehl identifizieren zu können.

Der Vergleich der Merkmalsvektoren kann einen individuellen Abgleich einzelner, bevorzugt aller von Merkmalsvektor umfassten Merkmale umfassen. Alternativ oder zusätzlich kann der Vergleich auf einem aus dem Merkmalsvektor abgeleiteten Merkmalsparameter basieren, welche Einzelmerkmale berücksichtigt. Das sich bei dem Vergleich ergebende Übereinstimmungsmaß für die Merkmalsvektoren oder Merkmalsparameter gibt an, welcher Sprachbefehl bzw. welche Befehlsklasse zugeordnet wird. Es wird, die Befehlsklasse zugeordnet, die die größte Ähnlichkeit bzw. eine Ähnlichkeit oberhalb eines festgelegten Schwellwertes hat.

Der Schwellwert für das festgelegte Ähnlichkeitsmaß kann automatisch oder durch eine Bedienperson voreingestellt werden. Es kann ferner von der für das Audiosignal erkannten spezifischen Merkmalskombination abhängen. Der Schwellwert kann eine Vielzahl von Einzelschwellwerten für individuelle Merkmale des Merkmalsvektors oder einen universellen Schwellwert berücksichtigend die Vielzahl der im Merkmalsvektor umfassten Einzelmerkmale darstellen.

In bevorzugten Ausführungen der Erfindung kann ein weiterer Computerlinguistik-Algorithmus für die Analyse der bestätigenden Benutzereingabe in Form eines Audiosignals ebenfalls als weitere trainierte Funktion, also ein weiterer trainierter Algorithmus des maschinellen Lernens ausgebildet sein. Die weitere trainierte Funktion kann, so wie mit Bezug zur ersten trainierten Funktion beschrieben, darauf trainiert sein, insbesondere mit einer ersten Gruppe neuronaler Netz-Schichten, die als Eingabedaten einen dem auf die Bestätigung gerichteten Audiosignal entsprechenden Datenstrom verwenden, akustische Merkmale wie Frequenz, Amplitude, Modulation oder dergleichen zu extrahieren und bspw. in Form eines weiteren akustischen Merkmalsvektors insbesondere einer zweiten Gruppe neuronaler Netz-Schichten bereitzustellen. Die weitere trainierte Funktion kann ferner darauf trainiert sein, insbesondere mit der zweiten Gruppe neuronaler Netz-Schichten Schlüsselworte oder Befehlstrigger aus dem Merkmalsvektor abzuleiten. Die zweite Gruppe neuronaler Netz-Schichten dient also dazu, dem akustischen Merkmalsvektor ein eindeutiges Schlüsselwort zuzuweisen. Dazu kann in Bezug auf einen erkannten Sprachbefehl bzw. eine Befehlsklasse eine geringe Menge von Schlüsselwörtern hinterlegt sein, bspw. ein oder zwei Schlüsselwörter, die für eine Bestätigung eines Sprachbefehls erkannt werden müssen, bspw. kann genau ein Schlüsselwort hinterlegt sein, insbesondere dann, wenn die (akustische) Ausgabe der Aufforderung zur Bestätigung des ersten Sprachbefehls auch eine Angabe umfasst, wie, bspw. mit welchem Schlüsselwort die Bestätigung erfolgen soll, oder auch zwei oder drei Schlüsselworte oder eine Wortfolge.

Schlüsselworte sind insbesondere kurze Worte oder Wortfolgen mit maximal drei oder vier Silben. Für die Schlüsselworte der einzelnen Befehlsklassen können ebenfalls spezifische akustische Merkmalsvektoren hinterlegt sein. Das neuronale Netz eines weiteren Computerlinguistik-Algorithmus kann entsprechend darauf trainiert sein, insbesondere mittels der zweiten Gruppe neuronaler Netz-Schichten, einen Vergleich zwischen dem aus dem auf die Bestätigung gerichteten Audiosignal extrahierten Merkmalsvektor und den hinterlegten Merkmalsvektoren entsprechend der jeweils zugewiesenen Schlüsselworte durchzuführen. Der Vergleich kann dabei so ausgeführt werden, wie mit Bezug zur ersten trainierten Funktion bereits beschrieben. Wird ein Schlüsselwort in einem auf die Bestätigung des ersten Sprachbefehls gerichteten Audiosignal erkannt und damit ein vorgegebenes Inhaltskriterium erfüllt, wird ein diese Übereinstimmung anzeigendes Verifikationssignal erzeugt, wie eingangs beschrieben. Das Verifikationssignal kann ferner auf den ersten bestätigten Sprachbefehl gerichtete zusätzliche Sprachsteuerinformationen umfassen, sofern diese von der Bedienperson erfragt und eingegeben wurden. Ermittelt die weitere trainierte Funktion keine Übereinstimmung mit einem der hinterlegten Schlüsselworte (Inhaltskriterium nicht erfüllt), wird ein Verifikationssignal erzeugt, das angibt, dass der erste Sprachbefehl nicht bestätigt wurde.

Während die erste trainierte Funktion darauf trainiert ist, einen möglichst breiten und vielseitigen Satz von Audiosignalen umfassend menschliche Sprache als einen Sprachbefehl auf einer vorab definierten, insbesondere großen Menge von Befehlsklassen zu erkennen, ist die weitere trainierte Funktion darauf trainiert, einige wenige Schlüsselworte in Audiosignalen zu identifizieren.

Im Übrigen wird für die weitere trainierte Funktion auf die Beschreibung der ersten trainierten Funktion verwiesen.

In weiteren, besonders bevorzugten Ausführungen der vorliegenden Erfindung umfasst das Ermitteln des Verifikationssignals folgendes:

- Analysieren des Audiosignals zum Bereitstellen eines zweiten Sprachanalyseergebnisses,
- Erkennen eines zweiten Sprachbefehls basierend auf dem zweiten Sprachanalyseergebnis,
- Vergleichen des ersten und des zweiten Sprachbefehls, wobei das Verifikationssignal den ersten Sprachbefehl bestätigt, wenn der erste Sprachbefehl und der zweite Sprachbefehl ein Übereinstimmungskriterium erfüllen.

In dieser Ausführung wird das Audiosignal ein zweites Mal, insbesondere unabhängig vom ersten Analyseschritt analysiert, um ein zweites Sprachanalyseergebnis generiert, um darauf basierend einen zweiten Sprachbefehl bereitzustellen, der dann mit dem ersten Sprachbefehl verglichen wird. Die auf das Ableiten des zweiten Sprachbefehls gerichteten Schritte sowie der Vergleich des ersten und des zweiten Sprachbefehls bilden also in Ausführungen der Erfindung ebenfalls einen P-Pfad aus, der vorteilhaft ohne eine weitere Nutzerinteraktion auskommt. Die auf das Ableiten des zweiten Sprachbefehls gerichteten Schritte sowie der Vergleich des ersten und des zweiten Sprachbefehls können zumindest teilweise zeitlich parallel zum Ableiten des ersten Sprachbefehls erfolgen. Bspw. kann das erfasste Audiosignal zeitgleich dem ersten und dem zweiten Analyseschritt zugeführt werden. Alternativ können diese Schritte zum Ermitteln des zweiten Sprachbefehls zeitlich nach dem Ableiten des ersten Sprachbefehls durchgeführt werden, dann insbesondere getriggert durch das Zuordnen des ersten Sprachbefehls zu der Sicherheitsklasse umfassend die erstfehlersicheren Sprachbefehle oder einer anderen Sicherheitsklasse mit hoher Sicherheitsstufe.

Das Verifikationssignal kann in einer vorteilhaften Ausführung also auf einem Vergleich zwischen dem ersten und dem zweiten Sprachbefehl basieren. Wird eine Übereinstimmung zwischen dem ersten und dem zweiten Sprachbefehl bzw. den jeweils zugeordneten Befehlsklassen ermittelt, wird eine den ersten Sprachbefehl bestätigende Verifikationsinformation für das Verifikationssignal erzeugt. Das Ermitteln einer Übereinstimmung zwischen erstem und zweitem Sprachbefehl entspricht dabei einem erfindungsgemäßen Prüfschritt bzw. Bestätigungszyklus.

Zusätzlich kann in Ausführungen der Erfindung weitere Bestätigungszyklen vorgesehen sein. Entsprechend kann vorgesehen sein, dass zusätzlich zu der automatischen Ermittlung der Übereinstimmung zwischen erstem und zweitem Sprachbefehl noch wenigstens ein Bestätigungszyklus, wie oben beschrieben, durchlaufen werden muss, in welchem eine Bedienperson bevorzugt mittels automatischer Sprachausgabe aufgefordert wird, eine bestätigende Benutzereingabe zu tätigen, um den ersten bzw. zweiten Sprachbefehl als das gewünschte Kommando zu verifizieren.

Das Verifikationssignal basiert in Ausführungen also auf dem Erfüllen eines Übereinstimmungskriteriums von erstem und zweitem Sprachbefehl und ggf. der Erfüllung eines Inhalts- und/oder Zeitkriteriums. Diese Implementierungen sind besonders für Sprachbefehle hoher Sicherheitsstufen geeignet.

Gemäß einer bevorzugten Ausführung umfasst das (zweite) Analysieren ein Anwenden eines zweiten Computerlinguistik-Algorithmus umfassend eine zweite trainierte Funktion auf das Audiosignal. Bevorzugt umfasst auch das Erkennen des zweiten Sprachbefehls basierend auf dem zweiten Sprachanalyseergebnis ein Anwenden des zweiten Computerlinguistik-Algorithmus. Erste und zweite trainierte Funktion sind erfindungsgemäße verschiedene trainierte Funktionen.

Speziell ist die zweite trainierte Funktion in Implementierungen dazu ausgebildet, nur sicherheitskritische Sprachbefehle in dem Audiosignal zu identifizieren. In diesem Sinne ist die zweite trainierte Funktion bzw. der zweite Computerlinguistik-Algorithmus in bevorzugten Ausführungen spezifisch für die Sprachbefehle der sicherheitskritischen Sicherheitsklasse umfassend die erstfehlersicheren Sprachbefehle.

Das Analysieren mit dem zweiten Computerlinguistik-Algorithmus kann ebenfalls ein Verfahren zur Verarbeitung natürlicher Sprache anwenden. Der zweite Computerlinguistik-Algorithmus kann auf das Audiosignal bzw. die Spracheingabe angewandt werden. Dabei kann eine Umwandlung der in natürlicher Sprache formulierten Spracheingabe zur Bereitstellung des zweiten Sprachanalyseergebnisses in Text, also in strukturierte Sprache, mittels eines Sprache-zu-Text (Software-)Moduls umfassen. Anschließend kann eine weitere Analyse zur Bereitstellung des zweiten Sprachanalyseergebnisses, beispielsweise mittels latenter semantischer Analyse (engl. latent semantic indexing, kurz LSI), dem Text eine Bedeutung beimessen. Auch hier wird das Audiosignal als Ganzes analysiert. Es kann ebenfalls das Erkennen von einzelnen Worten oder Wortgruppen und/oder einer Syntax umfasst sein. Die Beziehung/Stellung von Worten oder Wortgruppen zu vorher oder später in dem Audiosignal erscheinenden Worten oder Wortgruppen kann ebenfalls berücksichtigt werden. Derart realisiert die Erfindung ein Verstehen der in der Spracheingabe enthaltenen natürlichen Sprache (engl.: natural language understanding, NLU).

Der Schritt des Erkennens des zweiten Sprachbefehls basierend auf dem zweiten Sprachanalyseergebnis ist ebenfalls darauf gerichtet, der in der Spracheingabe erkannten Bedeutung des umfassten Textes einen eindeutigen Sprachbefehl zuzuordnen bzw. mit einem vordefinierten Satz sicherheitskritischer, insbesondere erstfehlersicherer Sprachbefehle in Verbindung zu bringen. Jeder sicherheitskritische Sprachbefehl ist dabei repräsentativ für eine definierte, sicherheitskritische Aktion, Operation oder Bewegung, die basierend auf dem Sprachbefehl automatisch von der medizinischen Vorrichtung ausgeführt werden soll. Das Erkennen des zweiten Sprachbefehls umfasst also insbesondere das Erkennen eines Sprachbefehls der sicherheitskritischen Sicherheitsklasse umfassend erstfehlersichere Sprachbefehle.

Auch der Schritt des Erkennens des zweiten Sprachbefehls kann durch Anwenden des zweiten Computerlinguistik-Algorithmus ausgeführt werden. Der Schritt des Erkennens des zweiten Sprachbefehls folgt dabei bevorzugt dem Erstellen eines Transkripts (strukturierte Sprache) des Sprachsignals bzw. dem auf dem Transkript basierenden Erstellen eines semantischen Analyseergebnisses. Beides kann in dem zweiten Sprachanalyseergebnis umfasst sein.

Jede sicherheitskritische Sicherheitsklasse kann eine Vielzahl von verschiedenen sicherheitskritischen Sprachbefehlen umfassen. Erkennt der zweite Computerlinguistik-Algorithmus keinen sicherheitskritischen Sprachbefehl, erfolgt kein Vergleich des ersten und des zweiten Sprachbefehls und es wird ein Verifikationssignal erstellt, das anzeigt, dass kein zweiter Sprachbefehl vorliegt. Das Verfahren wird abgebrochen und der erste Sprachbefehl wird verworfen.

Erkennt der zweite Computerlinguistik-Algorithmus einen sicherheitskritischen Sprachbefehl, erfolgt ein Vergleich des ersten und des zweiten Sprachbefehls. Zeigt der Vergleich keine Überstimmung der beiden Sprachbefehle, wird ein Verifikationssignal erzeugt, das den ersten Sprachbefehl nicht bestätigt und dieser wird ebenfalls verworfen. Zeigt der Vergleich eine Überstimmung der beiden Sprachbefehle, wird ein Verifikationssignal erzeugt, die den ersten Sprachbefehl bestätigt.

Der Vergleich dient der Ermittlung eines Übereinstimmungsmaßes zwischen den beiden erkannten Sprachbefehlen. Nur wenn bspw. eine Identität zwischen erstem und zweiten Sprachbefehl festgestellt wird, ist das Übereinstimmungskriterium erfüllt. In anderen Ausführungen kann auch ein geringeres Übereinstimmungsmaß ausreichend sein, um das vorab festgelegte Übereinstimmungskriterium zu erfüllen. Entsprechend können erfindungsgemäß insbesondere von dem zweiten Sprachbefehl abhängige Schwellwerte für die Menge der möglichen zweiten Sprachbefehle hinterlegt sein, wobei die Schwellwerte ein jeweiliges Sicherheitslevel repräsentieren können.

Auch die zweite trainierte Funktion bzw. der zweite trainierte Algorithmus des maschinellen Lernens umfasst ein neuronales Netz, welches im Wesentlichen wie mit Bezug zur ersten trainierten Funktion beschrieben, ausgebildet sein kann.

Gemäß Ausführungen der Erfindung ist auch das zweite neuronale Netz ein trainiertes Netz. Das Training des neuronalen Netzes wird bevorzugt im Sinne eines 'überwachten Lernens' basierend auf Trainingsdaten eines Trainingsdatensatzes, nämlich bekannten Paaren von Eingabe- und Ausgabedaten durchgeführt, wie oben beschrieben. Erfindungsgemäß wird also in Ausführungen das zweite neuronale Netz, also die zweite trainierte Funktion des zweiten Computerlinguistik-Algorithmus in einer Trainingsphase darauf trainiert, das erfasste Audiosignal zu analysieren und einen zweiten Sprachbefehl entsprechend einer der sicherheitskritischen Sicherheitsklassen, insbesondere der die erstfehlersicheren Sprachbefehle umfassenden Sicherheitsklasse daraus zu erkennen. Der zweite Sprachbefehl kann in Ausführungen den Ausgabedaten der zweiten trainierten Funktion entsprechen. In anderen Ausführungen übernimmt die zweite trainierte Funktion auch den Schritt des Vergleichens des ersten und des zweiten Sprachbefehls.

Sicherheitskritische Sprachbefehle, die der zweite Computerlinguistik-Algorithmus erkennen soll, weisen eine charakteristische Merkmalskombination auf, also eine charakteristisches Frequenzmuster, Amplitude, Modulation, oder dergleichen auf, die sich insbesondere von der Merkmalskombination derjenigen Sprachbefehle unterscheidet, die der erste Computerlinguistik-Algorithmus erkennen soll. Sicherheitskritische, insbesondere erstfehlersichere Sprachbefehle können bspw. durch eine Mindest-Silbenanzahl gekennzeichnet sein, die bspw. bei drei oder mehr liegt. Oder sie weisen eineindeutige, also kaum verwechselbare phonetische Merkmale auf, sodass eine Ähnlichkeit zu anderen Sprachbefehlen oder allgemein anderen Spracheingaben und eine damit einhergehende Verwechslungsgefahr von vornherein minimiert ist.

Entsprechend lernt auch die zweite trainierte Funktion in der Trainingsphase, anhand einer aus dem Audiosignal extrahierten Merkmalskombination im Sinne des zweiten Sprachanalyseergebnisses einen der sicherheitskritischen Sprachbefehle zuzuordnen. Die Trainingsphase kann auch eine manuelle Zuordnung von Trainings-Eingabedaten in Form von Spracheingaben zu einzelnen Sprachbefehlen umfassen.

Wie bei der ersten trainierten Funktion kann auch bei der zweiten trainierten Funktion eine erste Gruppe der neuronalen Netz-Schichten auf das Extrahieren bzw. das Ermitteln der akustischen Merkmale eines Audiosignals und das Bereitstellen des zweiten Sprachanalyseergebnisses gerichtet sein. Es wird auf die vorherige Beschreibung verwiesen, die hier übertragen werden kann. Das zweite Sprachanalyseergebnis kann ebenfalls als Eingabe-Datensatz für eine zweite Gruppe neuronaler Netz-Schichten, dem ‚Classifier', dienen, die dem zweiten Sprachanalyseergebnis einen der sicherheitskritischen Sprachbefehle zuordnet. Die zweite trainierte Funktion kann vorteilhaft ausgebildet sein, dem zweiten Sprachbefehl eine Fehler-Ausgabe zuzuordnen, wenn kein sicherheitskritischen Sprachbefehl erkannt wurde. Erkennt die zweite trainierte Funktion keinen sicherheitskritischen Sprachbefehl, wird der erste Sprachbefehl im Sinne einer Sicherheit von Mensch und Gerät immer verworfen.

Auch hier können die einzelnen Funktionen alternativ von mehreren, insbesondere zwei eigenständigen neuronalen Netzen ausgeführt werden.

Das Klassifizieren des Audiosignals in einen der sicherheitskritischen, insbesondere erstfehlersicheren Sprachbefehle basierend auf dem zweiten Sprachanalyseergebnis kann ebenfalls auf einem Vergleich eines extrahierten, zweiten Merkmalsvektors des Audiosignals mit einer Vielzahl von für jeden sicherheitskritischen Sprachbefehl spezifischen und hinterlegten Merkmalsvektoren basieren. Für jeden sicherheitskritischen, insbesondere erstfehlersicheren Sprachbefehl ist in Ausführungen der Erfindung genau ein Merkmalsvektor hinterlegt, um den Sicherheitsanforderungen für sicherheitskritische Aktionen oder Bewegungen der medizinischen Vorrichtung Rechnung zu tragen. Der Vergleich der Merkmalsvektoren kann einen individuellen Abgleich einzelner, bevorzugt aller von einem Merkmalsvektor umfassten Merkmale umfassen. Alternativ oder zusätzlich kann der Vergleich auf einem aus dem jeweiligen Merkmalsvektor abgeleiteten Merkmalsparameter basieren, welche eine Untermenge oder alle Einzelmerkmale berücksichtigt. Das sich bei dem Vergleich ergebende Übereinstimmungsmaß für die Merkmalsvektoren oder Merkmalsparameter gibt an, welcher sicherheitskritische Sprachbefehl zugeordnet wird bzw., dass kein sicherheitskritischer Sprachbefehl erkannt wurde. Es wird der sicherheitskritische Sprachbefehl als zweiter Sprachbefehl zugewiesen, der die größte Ähnlichkeit bzw. eine Ähnlichkeit zum oberhalb eines festgelegten Schwellwertes hat. Im Übrigen wird auf die Ausführungen zu der ersten trainierten Funktion verwiesen.

Ein wesentlicher Unterschied zwischen der ersten und der zweiten trainierten Funktion kann also insbesondere in der Art bzw. dem Umfang der Trainingsdaten liegen. Während die erste trainierte Funktion mit einem ersten Trainingsdatensatz trainiert wird, der einen breiten und vielseitigen Satz von Audiosignalen umfassend menschliche Sprache umfasst, der eine breite Vielzahl an verschiedenen Sprachbefehlen entsprechend einer breiten Vielzahl von Befehlsklassen und weitere allgemeine, keiner Befehlsklasse zuordenbaren Spracheingaben umfassen kann, wird die zweite trainierte Funktion mit einem zweiten Trainingsdatensatz trainiert, der auf spezifische, konkret sicherheitskritische, insbesondere erstfehlersichere, also phonetisch eindeutige, nur schwer verwechselbare Sprachbefehle beschränkt ist. Insbesondere ist in Ausführungen der zweite Trainingsdatensatz eine Untermenge des ersten Trainingsdatensatzes, wobei die Untermenge in Ausführungen nur erstfehlersichere Sprachbefehle betreffen kann. Insofern kann erfindungsgemäß die zweite trainierte Funktion mittels eines kleinen Trainings-Wortschatz (engl. small vocabulary) und im Gegensatz dazu die erste trainierte Funktion mit einem großen Trainings-Wortschatz (engl. large vocabulary) trainiert werden.

Ein weiterer Unterschied zwischen erster und zweiter trainierter Funktion kann in der Ausbildung der Verifikationsfunktion bzw. der Klassifizierungsfunktion liegen, die bevorzugt mittels der zweiten Gruppe neuronaler Netz-Schichten ausgeführt wird. Während die erste trainierte Funktion ausgebildet ist, eine Spracheingabe in eine breite Vielzahl verschiedener Sprachbefehle entsprechend einer großen Anzahl verschiedener Kategorien zu klassifizieren, ist die zweite trainierte Funktion ausgebildet, eine Spracheingabe in nur eine kleine Menge sicherheitskritischer Sprachbefehle entsprechend einer geringen Anzahl von Kategorien zu klassifizieren.

Insbesondere unterscheiden sich erfindungsgemäß die erste trainierte Funktion und die zweite trainierte Funktion in der Art bzw. dem Typ des jeweils eingesetzten neuronalen Netzes. Derart kann bspw. das Risiko für das Auftreten ähnlicher systematischer Fehler bei der Sprachbefehlserkennung durch die erste trainierte Funktion und die zweite trainierte Funktion reduziert werden.

Besonders bevorzugt kann bei der ersten trainierten Funktion auf an sich bekannte und verfügbare Spracherkennungs-Algorithmen zurückgegriffen werden. In Implementierungen der Erfindung entspricht die zweite trainierte Funktion einem im Rahmen eines sicheren, bspw. herstellerseitigen Software-Entwicklungsprozesses erzeugten Spracherkennungs-Algorithmus.

Während die erste trainierte Funktion bspw. als ein Feedforward-Netz ausgebildet sein kann, kann die zweite trainierte Funktion bspw. als rekurrentes bzw. rückgekoppeltes Netz ausgebildet sein, bei dem Knoten einer Schicht auch mit sich selbst oder mit anderen Knoten derselben und/oder wenigstens einer vorangegangenen Schicht verknüpft sind.

Gemäß einigen Implementierungen kann der erste Computerlinguistik-Algorithmus als sog. Front-End-Algorithmus implementiert sein, der z.B. in einer lokalen Recheneinheit der medizinischen Vorrichtung oder in einem lokalen Spracherkennungsmodul, gehostet ist. Als Front-End kann die Verarbeitung insbesondere gut in Echtzeit erfolgen, sodass das Ergebnis praktisch ohne nennenswerte Zeitverzögerung erhalten werden kann. Entsprechend kann der zweite Computerlinguistik-Algorithmus als sog. Back-End-Algorithmus implementiert sein, der z.B. in einer entfernten Rechenleinrichtung, wie etwa einem reellen Server-basierten Rechensystem oder einem virtuellen Cloud-Rechensystem, gehostet ist. In einer Back-End-Implementierung können insbesondere komplexe Analysealgorithmen eingesetzt werden, die eine hohe Rechenleistung erfordern. Entsprechend kann das Verfahren ein Übermitteln des Audiosignals an eine entfernte Recheneinrichtung und ein Empfangen ein oder mehrerer Analyseergebnisse von der entfernten Recheneinrichtung umfassen. In alternativen Implementierungen kann auch der zweite Computerlinguistik-Algorithmus als Front-End-Algorithmus implementiert sein. Umgekehrt kann auch der erste Computerlinguistik-Algorithmus als Back-End-Algorithmus implementiert sein.

Gemäß einer Vielzahl bevorzugter Implementierungen der Erfindung umfasst ein Analysieren des Audiosignals

- ein Tokenisieren zur Segmentierung von Buchstaben, Wörtern und/oder Sätzen innerhalb des Audiosignals und der erste und/oder der zweite Sprachbefehl basierend auf einer ersten Tokenisierungsinformation und/oder einer zweiten Tokenisierungsinformation erkannt werden,
und/oder
- eine semantische Analyse des Audiosignals und der erste und der zweite Sprachbefehl basierend auf einer ersten semantischen Information und einer zweiten semantischen Information erkannt werden.

Auch auf die auf die Bestätigung des ersten Sprachsteuerbefehls gerichtete Benutzereingabe in Form einer Spracheingabe kann mittels des weiteren Computerlinguistik-Algorithmus ein Tokenisieren und/oder eine semantische Analyse angewandt werden, wie sie im Folgenden genauer beschrieben werden.

Tokenisierung bezeichnet in der Computerlinguistik die Segmentierung eines Textes in Einheiten der Buchstaben-, Wort- und Satzebene. Gemäß einigen Implementierungen kann das Tokenisieren ein Umwandeln der im Audiosignal enthaltenen Sprache in Text umfassen. Mit anderen Worten kann ein Transskript erstellt werden, das dann tokenisiert wird. Dazu kann auf eine Vielzahl an sich bekannter Verfahren zurückgegriffen werden, die z.B. auf einer Verwendung einer Formantanalyse, von Hidden-Markov-Modellen, neuronalen Netzen, elektronischen Wörterbüchern und/oder Sprachmodellen beruhen. Bevorzugt wird dieser Analyseschritt mittels der ersten, zweiten und/oder der weiteren trainierten Funktion, wie eingangs beschrieben, ausgeführt.

Das erste und/oder zweite Sprachanalyseergebnis kann eine erste bzw. zweite Tokenisierungsinformation umfassen. Durch die Verwendung der Tokenisierungsinformation kann die Struktur einer Spracheingabe für die Ermittlung eines Sprachbefehls bzw. allgemein einer Nutzerintention berücksichtigt werden.

Gemäß einigen Implementierungen umfasst ein erfindungsgemäßer Analyseschritt des Audiosignals eine semantische Analyse des Audiosignals zur Ermittlung eines Sprachbefehls der Bedienperson. Entsprechend kann das erste und/oder zweite Sprachanalyseergebnis eine entsprechende semantische Information umfassen.

Die semantische Analyse stellt mit anderen Worten darauf ab, die Bedeutung der Spracheingabe der Bedienperson zu erschlie-ßen. Insbesondere kann die semantische Analyse einen vorgelagerten Spracherkennungsschritt (speech to text) und oder einen Tokenisierungsschritt umfassen.

Gemäß einigen Implementierungen zeigt die semantische Information an, ob das Audiosignal ein oder mehrere Nutzerintentionen enthält oder nicht. Die Nutzerintention kann dabei insbesondere eine auf einen oder mehrere in Frage kommende Sprachbefehle gerichtete Spracheingabe der Bedienperson sein. Die Sprachbefehle können dabei insbesondere zur Steuerung der medizinischen Vorrichtung relevante Sprachbefehle sein. Gemäß einigen Implementierungen indiziert bzw. enthält die semantische Information wenigstens eine Eigenschaft einer in dem Audiosignal enthaltenen Nutzerintention. Mit der semantischen Analyse werden also bestimmte akustische Charakteristika bzw. Eigenschaften aus der Spracheingabe extrahiert, die für die Bestimmung eines Sprachbefehls berücksichtigt werden bzw. relevant sein können.

Gemäß einem weiteren Aspekt stellt die Erfindung eine Sprachsteuervorrichtung zur Sprachsteuerung einer medizinischen Vorrichtung bereit. Die Sprachsteuervorrichtung umfasst wenigstens eine Schnittstelle zum Erfassen eines Audiosignals enthaltend eine auf die Steuerung der Vorrichtung gerichtete Spracheingabe einer Bedienperson. Die Sprachsteuervorrichtung umfasst ferner wenigstens ein Auswerteeinheit, die ausgebildet ist, das Audiosignal zu analysieren und ein erstes Sprachanalyseergebnis bereitzustellen, einen ersten Sprachbefehl basierend auf dem ersten Sprachanalyseergebnis zu erkennen, den ersten Sprachbefehl zu einer Sicherheitsklasse zuzuordnen, wobei eine Sicherheitsklasse für sicherheitskritische Sprachbefehle vorgesehen ist, ein Verifikationssignal für den ersten Sprachbefehl zu ermitteln und ggf. zur Weiterverarbeitung bereitzustellen. Die Sprachsteuervorrichtung umfasst weiter eine Steuereinheit, die ausgebildet ist, ein Steuersignal zur Steuerung der medizinischen Vorrichtung basierend auf dem ersten Sprachbefehl und dem Verifikationssignal zu erzeugen, sofern der erste Sprachbefehl anhand der Verifikationsinformation bestätigt wurde, wobei das Steuersignal geeignet ist, die medizinische Vorrichtung entsprechend dem ersten Sprachbefehl zu steuern. Die Sprachsteuervorrichtung umfasst auch eine Schnittstelle zur Eingabe des Steuersignals in die medizinische Vorrichtung.

Eine medizinische Vorrichtung im Sinne der Erfindung ist insbesondere eine gegenständliche medizinische Vorrichtung. Die medizinische Vorrichtung wird typischerweise zur Behandlung und/oder Untersuchung eines Patienten eingesetzt. Die medizinische Vorrichtung kann insbesondere dazu ausgebildet sein, eine medizinische Prozedur durchzuführen und/oder zu unterstützen. Die medizinische Prozedur kann eine bildgebende und/oder interventionelle und/oder therapeutische Prozedur aber auch eine Überwachung eines Patienten umfassen. Insbesondere kann die medizinische Vorrichtung eine bildgebende Modalität umfassen, wie beispielsweise ein Magnetresonanzgerät, ein Einzelphotonenemissionstomographie-Gerät (SPECT-Gerät), ein Positronen-Emissions-Tomographie-Gerät (PET-Gerät), ein Computertomograph, ein Ultraschall-Gerät, ein Röntgengerät oder ein als C-Bogen-Gerät ausgebildetes Röntgengerät. Die bildgebende Modalität kann auch ein kombiniertes medizinisches Bildgebungsgerät sein, welches eine beliebige Kombination aus mehreren der genannten bildgebenden Modalitäten umfasst. Ferner kann die medizinische Vorrichtung eine Interventions- und/oder Therapievorrichtung aufweisen, wie etwa eine Biopsievorrichtung, eine Strahlen- bzw. Radiotherapievorrichtung zur Bestrahlung eines Patienten, und/oder eine Eingriffsvorrichtung zur Durchführung eines, insbesondere minimalinvasiven, Eingriffs. Gemäß weiteren Implementierungen umfasst die medizinische Vorrichtung zusätzlich oder alternativ Patientenüberwachungsmodule, wie etwa ein EKG-Gerät, und/oder ein Patienten-Pflegegerät umfassen, wie etwa ein Beatmungsgerät, ein Infusionsgerät und/oder ein Dialysegerät. Die Behandlung und/oder Untersuchung und/oder Überwachung des Patienten mittels der medizinischen Vorrichtung wird in Ausführungen typischerweise von einer Bedienperson, beispielsweise Pflegepersonal, technischem Personal, Röntgenassistenzpersonal oder Ärztinnen, unterstützt bzw. gesteuert.

Die wenigstens eine Auswerteeinheit sowie die Steuereinheit kann als eine oder mehrere zentrale und/oder dezentrale Recheneinheiten ausgebildet sein. Die Recheneinheit(en) kann/können jeweils einen oder mehrere Prozessoren aufweisen. Ein Prozessor kann als zentrale Verarbeitungseinheit (CPU/GPU) ausgebildet sein. Insbesondere kann die wenigstens eine Auswerteeinheit sowie die Steuereinheit jeweils als Teil oder Modul einer, durch Spracheingabe zu steuernden, medizinischen Vorrichtung implementiert sein. In Implementierungen kann die wenigstens eine Auswerteeinheit als Submodul der Steuereinheit ausgebildet sein oder umgekehrt. Alternativ kann die wenigstens eine Auswerteeinheit als lokaler oder Cloud-basierter Verarbeitungsserver implementiert sein. Ferner kann die wenigstens eine Auswerteeinheit ein oder mehrere virtuelle Maschinen umfassen.

In bevorzugten Implementierungen umfasst die Sprachsteuervorrichtung zwei separate Auswerteeinheiten, die jeweils wie oben beschrieben und bevorzugt auf separater Hardware bzw. in verschiedenen Software-Modulen implementiert sind. Dabei ist eine erste Auswerteinheit ausgebildet, die folgenden erfindungsgemäßen Schritte auszuführenden: das Audiosignal zu analysieren und ein erstes Sprachanalyseergebnis bereitzustellen, einen ersten Sprachbefehl basierend auf dem ersten Sprachanalyseergebnis zu erkennen und den ersten Sprachbefehl zu einer Sicherheitsklasse zuzuordnen. Die zweite Auswerteinheit ist ausgebildet eine Verifikationsinformation für den ersten Sprachbefehl zu ermitteln, sofern dieser einer Sicherheitsklasse für sicherheitskritische Sprachbefehle zugeordnet wurde, und ein Verifikationssignal basierend auf der Verifikationsinformation bereitzustellen.

Derart ist die vorliegende Erfindung ausgebildet, die klassische Control-Protect (CP) -Struktur eines erstfehlersicheren Steuerungssystems für Steuerbefehle in Form von Spracheingabe ab, in welchem die erste Auswerteinheit einen Teil des Steuer-Pfades (C-Pfad) und die zweite Auswerteinheit den Prüf-Pfad (P-Pfad) ausbildet.

Die Schnittstelle der Sprachsteuervorrichtung kann allgemein zum Datenaustausch zwischen der Sprachsteuerungsvorrichtung und weiteren Komponenten und oder zum Datenaustausch von Komponenten oder Modulen der Sprachsteuervorrichtung untereinander ausgebildet sein. Die Schnittstelle kann insofern in Form von einer oder mehreren einzelnen Datenschnittstellen implementiert sein, welche ein Hardware- und/oder Software-Interface, z.B. einen PCI-Bus, eine USB-Schnittstelle, eine Fire-Wire-Schnittstelle, eine ZigBee- oder eine Bluetooth-Schnittstelle aufweisen können. Die Schnittstelle kann ferner eine Schnittstelle eines Kommunikationsnetzwerks aufweisen, wobei das Kommunikationsnetzwerk ein Local Area Network (LAN), beispielsweise ein Intranet oder ein Wide Area Network (WAN) aufweisen kann. Entsprechend können die ein oder mehreren Datenschnittstellen eine LAN-Schnittstelle oder eine Wireless LAN-Schnittstelle (WLAN oder Wi-Fi) aufweisen. Die Schnittstelle kann ferner zur Kommunikation mit der Bedienperson über eine Benutzerschnittstelle ausgebildet sein. Entsprechend kann die Schnittstelle dazu ausgebildet sein, Sprachbefehle über die Benutzerschnittstelle anzuzeigen und diesbezügliche Nutzereingaben über die Benutzerschnittstelle zu empfangen. Insbesondere kann die Schnittstelle eine akustische Eingabevorrichtung zur Registrierung des Audiosignals und in Ausführungen eine akustische Ausgabevorrichtung zur Ausgabe eines eine Aufforderung zur Bestätigung des ersten Sprachbefehls umfassenden Audiosignals umfassen.

Die Vorteile der vorgeschlagenen Vorrichtung entsprechen im Wesentlichen den Vorteilen des vorgeschlagenen Verfahrens. Merkmale, Vorteile oder alternative Ausführungsformen/Aspekte können ebenso auf die anderen beanspruchten Gegenstände übertragen werden und umgekehrt.

Gemäß einem weiteren Aspekt stellt die Erfindung ein medizinisches System umfassend die erfindungsgemäße Sprachsteuervorrichtung und eine medizinische Vorrichtung zur Durchführung einer medizinischen Prozedur bereit.

Die Erfindung betrifft in einem weiteren Aspekt ein Computerprogrammprodukt, das ein Programm umfasst und direkt in einen Speicher einer programmierbaren Recheneinheit ladbar ist und Programmmittel, bspw. Bibliotheken und Hilfsfunktionen, aufweist, um ein Verfahren zur Sprachsteuerung einer medizinischen Vorrichtung insbesondere gemäß den vorgenannten Implementierungen/Aspekten auszuführen, wenn das Computerprogrammprodukt ausgeführt wird.

Ferner betrifft die Erfindung in einem weiteren Aspekt ein computerlesbares Speichermedium, auf welchem lesbare und ausführbare Programmabschnitte gespeichert sind, um alle Schritte eines Verfahrens zur Sprachsteuerung einer medizinischen Vorrichtung gemäß den vorgenannten Implementierungen/Aspekten auszuführen, wenn die Programmabschnitte von einer Recheneinheit ausgeführt werden.

Das Computerprogrammprodukt kann dabei eine Software mit einem Quellcode, der noch kompiliert und gebunden oder der nur interpretiert werden muss, oder einen ausführbaren Softwarecode umfassen, der zur Ausführung nur noch in die Recheneinheit zu laden ist. Durch das Computerprogrammprodukt können die erfindungsgemäßen Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, einen entsprechenden Prozessor oder eine entsprechende Logikeinheit aufweisen, sodass die jeweiligen Verfahrensschritte effizient ausgeführt werden können.

Das Computerprogrammprodukt ist beispielsweise auf einem computerlesbaren Speichermedium gespeichert oder auf einem Netzwerk oder Server hinterlegt, von wo es in den Prozessor der jeweiligen Recheneinheit geladen werden kann, der mit der Recheneinheit direkt verbunden oder als Teil der Recheneinheit ausgebildet sein kann. Weiterhin können Steuerinformationen des Computerprogrammprodukts auf einem computerlesbaren Speichermedium gespeichert sein. Die Steuerinformationen des computerlesbaren Speichermediums können derart ausgebildet sein, dass sie bei Verwendung des Datenträgers in einer Recheneinheit ein erfindungsgemäßes Verfahren durchführen. Beispiele für computerlesbaren Speichermedium sind eine DVD, ein Magnetband oder ein USB-Stick, auf welchem elektronisch lesbare Steuerinformationen, insbesondere Software, gespeichert ist. Wenn diese Steuerinformationen von dem Datenträger gelesen und in eine Recheneinheit gespeichert werden, können alle erfindungsgemäßen Ausführungsformen/Aspekte der vorab beschriebenen Verfahren durchgeführt werden. So kann die Erfindung auch von dem besagten computerlesbaren Medium und/oder dem besagten computerlesbaren Speichermedium ausgehen. Die Vorteile der vorgeschlagenen Computerprogrammprodukte bzw. der zugehörigen computerlesbaren Medien entsprechen im Wesentlichen den Vorteilen der vorgeschlagenen Verfahren.

Weitere Besonderheiten und Vorteile der Erfindung werden aus den nachfolgenden Erläuterungen von Ausführungsbeispielen anhand von schematischen Zeichnungen ersichtlich. In diesem Zusammenhang genannte Modifikationen können jeweils miteinander kombiniert werden, um neue Ausführungsformen auszubilden. In unterschiedlichen Figuren werden für gleiche Merkmale die gleichen Bezugszeichen verwendet.

FIG 1   ein schematisches Blockdiagramm eines Systems zur Steuerung einer medizinischen Vorrichtung gemäß einer Ausführungsform,

FIG 2   ein weiteres Blockdiagramm eines Systems zur Steuerung einer medizinischen Vorrichtung gemäß einer weiteren Ausführungsform,

FIG 3   ein schematisches Ablaufdiagramm eines Verfahrens zur Steuerung einer medizinischen Vorrichtung gemäß einer Ausführungsform,

FIG 4   ein weiteres schematisches Ablaufdiagramm eines Verfahrens zur Steuerung einer medizinischen Vorrichtung gemäß einer Ausführungsform,

FIG 5   ein alternatives schematisches Ablaufdiagramm eines Verfahrens zur Steuerung einer medizinischen Vorrichtung gemäß einer Ausführungsform, und

FIG 6   ein neuronales Netz eines erfindungsgemäßen Computerlinguistik-Algorithmus in einer Ausführungsform.

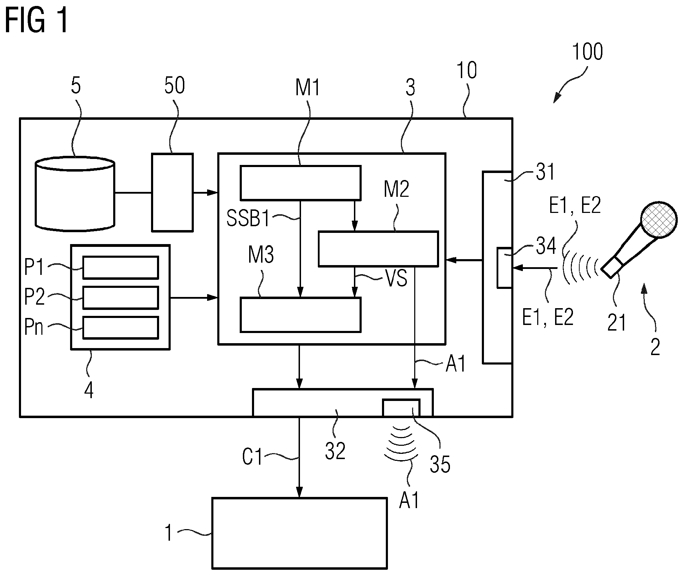

**Figur 1** zeigt schematisch eine funktionale Blockdarstellung eines Systems 100 zur Steuerung einer medizinischen Vorrichtung 1. Das System 100 umfasst die medizinische Vorrichtung 1, die zur Durchführung einer medizinischen Prozedur an einem Patienten ausgebildet ist. Die medizinische Prozedur kann eine bildgebende und/oder interventionelle und/oder therapeutische Prozedur umfassen. Das System umfasst ferner eine Sprachsteuervorrichtung 10.

Die medizinische Vorrichtung 1 kann eine bildgebende Modalität umfassen. Die bildgebende Modalität kann allgemein dazu ausgebildet sein, einen anatomischen Bereich eines Patienten abzubilden, wenn dieser in einen Erfassungsbereich der bildgebenden Modalität gebracht wird. Die bildgebende Modalität ist beispielsweise ein Magnetresonanzgerät, ein Einzelphotonenemissionstomographie-Gerät (SPECT-Gerät), ein Positronen-Emissions-Tomographie-Gerät (PET-Gerät), ein Computertomograph, ein Ultraschall-Gerät, ein Röntgengerät oder ein als C-Bogen-Gerät ausgebildetes Röntgengerät. Die bildgebende Modalität kann auch ein kombiniertes medizinisches Bildgebungsgerät sein, welches eine beliebige Kombination aus mehreren der genannten bildgebenden Modalitäten umfasst.

Ferner kann die medizinische Vorrichtung 1 eine Interventions- und/oder Therapievorrichtung aufweisen. Die Interventions- und/oder Therapievorrichtung kann allgemein dazu ausgebildet sein, eine interventionelle und/oder therapeutische medizinische Prozedur an dem Patienten durchzuführen. Beispielsweise kann die Interventions- und/oder Therapievorrichtung eine Biopsievorrichtung zur Entnahme einer Gewebeprobe, eine Strahlen- bzw. Radiotherapievorrichtung zur Bestrahlung eines Patienten, und/oder eine Eingriffsvorrichtung zur Durchführung eines, insbesondere minimalinvasiven, Eingriffs sein. Gemäß Ausführungsformen der Erfindung kann die Interventions- und/oder Therapievorrichtung automatisiert oder wenigstens teil-automatisiert sowie insbesondere robotergesteuert sein. Die Strahlen- bzw. Radiotherapievorrichtung kann beispielsweise einen medizinischen Linearbeschleuniger oder eine andere Strahlenquelle aufweisen. Beispielsweise kann die Eingriffsvorrichtung einen Katheterroboter, minimalinvasiven chirurgischen Roboter, einen Endoskopieroboter usw. aufweisen.

Gemäß weiterer Ausführungsformen kann die medizinische Vorrichtung 1 zusätzlich oder alternativ Einheiten und/oder Module aufweisen, welche die Durchführung einer medizinischen Prozedur unterstützen, wie etwa eine insbesondere wenigstens teil-automatisiert steuerbare Patientenlagerungsvorrichtung und/oder Überwachungsgeräte zur Überwachung eines Zustands des Patienten, wie etwa ein EKG-Gerät, und/oder ein Patientenversorgungsgerät, wie etwa ein Beatmungsgerät, ein Infusionsgerät und/oder ein Dialysegerät.

Ein oder mehrere Komponenten der medizinischen Vorrichtung 1 sollen gemäß Ausführungsformen der Erfindung durch eine oder mehrere Spracheingaben einer Bedienperson ansteuerbar sein. Dazu weist das System 100 eine Sprachsteuervorrichtung 10 umfassend eine Schnittstelle mit akustischer Eingabevorrichtung 2 auf.

Die akustische Eingabevorrichtung 2 dient zum Aufnehmen oder Erfassen eines Audiosignals E1, also zum Aufnehmen gesprochener Laute, die von einer Bedienperson des Systems 100 erzeugt werden. Die Eingabevorrichtung 2 kann beispielsweise als Mikrophon realisiert sein. Die Eingabevorrichtung 2 kann beispielsweise an der medizinische Vorrichtung 1 oder an anderer Stelle, wie in einem entfernten (engl. remote) Bedienraum, stationär angeordnet sein. Alternativ kann die Eingabevorrichtung 2 auch portabel realisiert sein, z.B. als Mikrophon eines Headsets, das von der Bedienperson mitführbar ist. In diesem Fall weist die Eingabevorrichtung 2 vorteilhaft einen Sender 21 zur drahtlosen Datenübertragung auf.

Die Sprachsteuervorrichtung 10 weist einen Eingang 31 zum Empfang von Signalen und einen Ausgang 32 zum Bereitstellen von Signalen auf. Der Eingang 31 und der Ausgang 32 können eine Schnittstelleneinrichtung der Sprachsteuervorrichtung 10 bilden. Die Sprachsteuervorrichtung 10 ist allgemein zur Durchführung von Datenverarbeitungsprozessen und zur Erzeugung elektrischer Signale eingerichtet.

Hierzu kann die Sprachsteuervorrichtung 10 wenigstens eine Recheneinheit 3 aufweisen. Die Recheneinheit 3 kann z.B. einen Prozessor, z.B. in Form einer CPU oder dergleichen umfassen. Die Recheneinheit 3 kann wenigstens eine zentrale Auswerteeinheit, z.B. als eine Auswerteeinheit mit einem oder mehreren Prozessoren ausgebildet sein. Die Recheneinheit 3 kann bevorzugt eine Steuereinheit umfassen, die ausgebildet ist, Steuersignale für die medizinische Vorrichtung zu erzeugen.

Die Recheneinheit 3 kann insbesondere zumindest teilweise als Steuerungsrechner (engl. System control) der medizinischen Vorrichtung 1 oder als ein Teil desselben ausgebildet sein. Die Recheneinheit 3 umfasst erfindungsgemäß Einheiten bzw. Module, die zur Ausführung einer insbesondere genormten Sicherheitsfunktion (auch SIL (engl. safety integrity level)-Einheiten) ausgebildet sind. Die genormte Sicherheitsfunktion dient dazu, ein Betriebsrisiko der medizinischen Vorrichtung 1 durch Ausführung eines fälschlich erkannten Steuerbefehls zu minimieren. Insbesondere ermöglicht die Sicherheitsfunktion die Absicherung eines Erstfehlers bei der maschinellen Erkennung eines Steuerbefehls aus einer Spracheingabe. Gemäß weiteren Implementierungen können Funktionalitäten und Komponenten der Recheneinheit 3 in einer dezentralisierten Weise über mehrere Recheneinheiten oder Steuermodule des Systems 100 verteilt sein.

Ferner weist die Sprachsteuervorrichtung 10 einen Datenspeicher 4 auf, und zwar insbesondere einen durch die Recheneinheit 3 lesbaren, nicht-flüchtigen Datenspeicher wie eine Festplatte, eine CD-ROM, eine DVD, eine Blu-Ray Disc, eine Diskette, einen Flash-Speicher oder dergleichen. Auf dem Datenspeicher 4 kann allgemein Software P1, P2, Pn gespeichert sein, die dazu eingerichtet ist, die Recheneinheit 3 zur Durchführung der Schritte eines Verfahrens zu veranlassen.

Wie in Figur 1 schematisch dargestellt ist, ist der Eingang 31 der Sprachsteuervorrichtung 10 mit der Eingabevorrichtung 2 verbunden. Der Eingang kann ebenfalls mit der medizinischen Vorrichtung 1 verbunden sein. Der Eingang 31 kann zur drahtlosen oder zur drahtgebundenen Datenkommunikation eingerichtet sein. Beispielsweise kann der Eingang 31 einen Bus-Anschluss aufweisen. Alternativ oder zusätzlich zu einem drahtgebundenen Anschluss kann der Eingang 31 auch eine Schnittstelle, z.B. einen Empfänger 34 zur drahtlosen Datenübertragung aufweisen. Beispielsweise kann, wie in Figur 1 dargestellt, der Empfänger 34 mit dem Sender 21 der Eingabevorrichtung 2 in Datenkommunikation stehen. Als Empfänger 34 kann beispielsweise eine WIFI Schnittstelle, eine Bluetooth-Schnittstelle oder dergleichen vorgesehen sein.

Der Ausgang 32 der Sprachsteuervorrichtung 10 ist einerseits mit der medizinische Vorrichtung 1 verbunden. Der Ausgang 32 kann zur drahtlosen oder zur drahtgebundenen Datenkommunikation eingerichtet sein. Beispielsweise kann der Ausgang 32 einen Bus-Anschluss aufweisen. Alternativ oder zusätzlich zu einem drahtgebundenen Anschluss kann der Ausgang 32 auch eine Schnittstelle zur drahtlosen Datenübertragung, bspw. zu einem Online-Modul OM1, aufweisen, beispielsweise eine WIFI Schnittstelle, eine Bluetooth-Schnittstelle oder dergleichen.

Der Ausgang 32 kann in Ausführungen zum Ausgeben eines Audiosignals A1, also zur akustischen Ausgabe gesprochener, natürlicher Sprache, die von einer Ausgabevorrichtung 35 erzeugt werden, ausgebildet sein. Die Ausgabevorrichtung 35 kann insbesondere ausgebildet sein, basierend auf einem Signal umfassend strukturierten Text maschinell eine Sprachausgabe zu erzeugen. Die Ausgabevorrichtung 35 kann bspw. als Lausprecher realisiert sein. Die Ausgabevorrichtung 35 kann ebenfalls an der medizinische Vorrichtung 1 oder an einem entfernten Bedienraum, stationär angeordnet sein. Alternativ kann die Ausgabevorrichtung 35 auch portabel realisiert sein, insbesondere kann sie mit der Eingabevorrichtung 2 kombiniert ausgebildet sein, z.B. als bereit genanntes Headset.

Die Sprachsteuervorrichtung 10 ist dazu eingerichtet, ein oder mehrere Steuersignale C1 zur Steuerung der medizinischen Vorrichtung 1 zu erzeugen und an dem Ausgang 32 bereitzustellen. Das Steuerkommando C1 veranlasst die medizinische Vorrichtung 1 zur Durchführung eines bestimmten Arbeitsschritts oder einer Sequenz von Schritten. Am Beispiel einer als MR-Gerät ausgeführten bildgebenden Modalität können sich solche Schritte beispielsweise auf die Durchführung einer bestimmten Scansequenz mit einer bestimmten Anregung von Magnetfeldern durch einen Erzeugerkreis des MR-Geräts beziehen. Ferner können sich solche Schritte auf ein Verfahren von verfahrbaren Systemkomponenten der medizinischen Vorrichtung 1 beziehen, wie etwa dem Verfahren einer Patientenlagerungsvorrichtung oder dem Verfahren von Emissions- oder Detektor-Komponenten einer bildgebenden Modalität. Die Schritte können sich insbesondere auch auf das Auslösen bzw. Starten einer Röntgenstrahlung beziehen.

Zur Bereitstellung des Steuersignals C1 kann die Recheneinheit 3 verschiedene Module M1-M3 aufweisen. Ein erstes Modul M1 entsprechend einer ersten Auswerteeinheit ist dazu ausgebildet, das Audiosignal E1 zu analysieren und basierend drauf ein erstes Sprachanalyseergebnis bereitzustellen, einen ersten Sprachbefehl SSB1 basierend auf dem ersten Sprachanalyseergebnis zu erkennen und den ersten Sprachbefehl SSB1 zu einer Sicherheitsklasse SK zuzuordnen. Hierfür kann das Modul M1 dazu ausgebildet sein, einen ersten Computerlinguistik-Algorithmus P1 auf das Audiosignal E1 anzuwenden. Insbesondere ist das Modul M1 ausgebildet, die Verfahrensschritte S20 bis S40 auszuführen.

Der erste Sprachbefehl SSB1 kann anschließend in ein weiteres Modul M2 entsprechend einer zweiten, von der ersten unabhängigen Auswerteeinheit eingegeben werden, wenn er einer Sicherheitsklasse SK umfassend sicherheitskritische Sprachbefehle zugeordnet wurde. Modul M2 ist dazu ausgebildet, ein Verifikationssignal VS zur Bestätigung des ersten Sprachbefehls SSB1, insbesondere gemäß wenigstens einer Ausführung des Verfahrensschritts S50, zu ermitteln. Dazu kann das Modul M2 ausgebildet sein, einen zweiten oder wenigstens einen weiteren Computerlinguistik-Algorithmus P2, Pn auf das Audiosignal E1 bzw. ein weiteres Audiosignal E2, welches ebenfalls über die Eingabevorrichtung 2 eingegeben werden und einer Benutzereingabe zur Bestätigung des ersten Sprachbefehls SSB1 entsprechen kann, anzuwenden. Der zweite Computerlinguistik-Algorithmus P2 kann dabei ausgebildet sein, einen zweiten Sprachbefehl SSB2 in dem Audiosignal E1 zu erkennen. Der weitere Computerlinguistik-Algorithmus Pn kann auch ausgebildet sein, wenigstens ein bestätigendes Schlüsselwort in wenigstens einem zweiten Audiosignal E2 zu erkennen. Dazu kann das zweite Modul M2 auch ausgebildet sein, zuvor basierend auf dem ersten Sprachbefehl SSB1 eine Sprachausgabe A1 zur Aufforderung der Bedienperson zur Bestätigung des ersten Sprachbefehls SSB1 zu erzeugen (wiederum unter Anwendung eines weiteren Computerlinguistik-Algorithmus), welche Aufforderung über die Ausgabevorrichtung 35 des Ausgangs 32 akustisch ausgegeben wird.

Das zweite Modul M2 ist insbesondere ausgebildet, basierend auf einem Vergleich zwischen erstem und zweitem Sprachbefehl SSB1, SSB2 oder basierend auf dem Erkennen eines vorab definierten Schlüsselwortes in dem wenigstens einen zweiten Audiosignal E2 ein Verifikationssignal VS umfassend eine Verifikationsinformation, bestätigend den ersten Sprachbefehl SSB1 zu erzeugen. Das Verifikationssignal VS kann bevorzugt auch den ersten Sprachbefehl SSB1 umfassen.

Das Verifikationssignal VS wird an ein drittes Modul M3 entsprechend einer Steuereinheit der Sprachsteuervorrichtung 10 gegeben. Modul M3 ist dazu ausgebildet, basierend auf dem ersten Sprachbefehl SSB1 bzw. dem Verifikationssignal VS ein oder mehrere Steuersignale C1 bereitzustellen, die dazu geeignet sind, die medizinische Vorrichtung 1 entsprechend dem ersten Sprachbefehl SSB1 zu steuern.

Gehört der erste Sprachbefehl SSB1 einer Sicherheitsklasse SK betreffend nicht sicherheitskritische Sprachbefehle an, wird das Modul M2 nicht aktiviert. Modul M1 gibt dann den erkannten ersten Sprachbefehl SSB1 bspw. direkt in das Modul M3 ein. Eine erfindungsgemäße Verifikation kann hier entfallen.

Die vorgenommene Unterteilung in Module M1-M3 dient dabei lediglich der einfacheren Erklärung der Funktionsweise der Recheneinheit 3 und ist nicht beschränkend zu verstehen. Die Module M1-M3 können dabei insbesondere auch als Computerprogrammprodukte oder Computerprogrammsegmente aufgefasst werden, welche bei der Ausführung in der Recheneinheit 3 ein oder mehrere der nachstehend beschriebenen Funktionen oder Verfahrensschritte realisieren.

Bevorzugt ist mindestens Modul M2 als eine SIL-Einheit zur Ausführung einer Sicherheitsfunktion ausgebildet. Die Sicherheitsfunktion ist erfindungsgemäß darauf gerichtet, den ersten erkannten Sprachbefehl SSB1 in wenigstens einer unabhängigen Verifikationsschleife zu prüfen bzw. zu bestätigen.

Entsprechend einer klassischen CP-Struktur bilden Modul M1 und Modul M3 zusammen einen Teil des C-Pfades aus, während Modul M2 einen Teil des P-Pfades ausbildet.

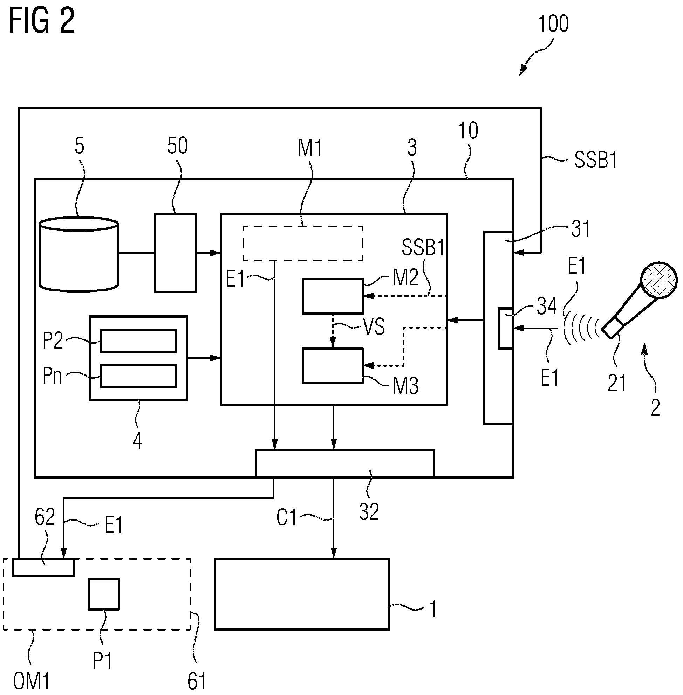

**Figur 2** zeigt schematisch eine funktionale Blockdarstellung eines Systems 100 zur Durchführung einer medizinischen Prozedur an einem Patienten gemäß einer weiteren Ausführungsform.

Die in Figur 2 gezeigte Ausführungsform unterscheidet sich von der in Figur 1 gezeigten Ausführungsform darin, dass die Funktionalitäten des Moduls M1 zumindest teilweise in ein Online-Modul OM1 ausgelagert sind. Ansonsten bezeichnen gleiche Bezugszeichen gleiche oder funktionsgleiche Komponenten.

Das Online-Modul OM1 kann auf einem Server 61 abgelegt sein, mit dem die Sprachanalysevorrichtung 10 über eine Internetverbindung und eine Schnittstelle 62 des Servers 61 in Datenaustausch treten kann. Die Sprachsteuervorrichtung 10 kann entsprechend dazu ausgebildet sein, dem Online-Modul OM1 das Audiosignal E1 zu übermitteln. Das Online-Modul OM1 kann dazu ausgebildet sein basierend auf dem Audiosignal E1 einen ersten Sprachbefehl SSB1 und ggf. die dazugehörige Sicherheitsklasse ermitteln und der Sprachsteuervorrichtung 10 zurückzugeben. Entsprechend kann das Online-Spracherkennungsmodul OM1 dazu ausgebildet sein, den ersten Computerlinguistik-Algorithmus P1 in einem geeigneten Online-Speicher verfügbar zu machen. Das Online-Modul OM1 kann dabei als zentralisierte Einrichtung aufgefasst werden, welche Spracherkennungsdienste für mehrere, insbesondere lokale Clients bereitstellt (die Sprachsteuervorrichtung 10 kann in diesem Sinne als lokaler Client aufgefasst werden). Die Verwendung eines zentralen Online-Moduls OM1 kann dahingehend vorteilhaft sein, dass leistungsfähigere Algorithmen angewandt und mehr Rechenleistung aufgebracht werden können.

In alternativen Implementierungen kann das Online-Spracherkennungsmodul OM1 auch "lediglich" Das erste Sprachanalyseergebnis zurückgeben. Das erste Sprachanalyseergebnis kann dann bspw. maschinen-verwertbaren Text enthalten, in den das Audiosignal E1 umgewandelt wurde. Auf Grundlage dessen kann das Modul M1 der Recheneinheit 3 den ersten Sprachbefehl SSB1 identifizieren. Eine solche Ausgestaltung kann dann von Vorteil sein, wenn die Sprachbefehle SSB1 von den Gegebenheiten der medizinischen Vorrichtung 1 abhängen, auf die das Online-Modul OM1 keinen Zugriff hat und/oder für deren Berücksichtigung das Online-Modul OM1 nicht vorbereitet wurde. Man bedient sich dann der Leistungsfähigkeit des Online-Moduls OM1 zur Erstellung eines ersten Sprachanalyseergebnisses, bestimmt die Sprachbefehle aber ansonsten innerhalb der Recheneinheit 3.

Gemäß einer weiteren, nicht gezeigten Abwandlung können umgekehrt aber auch weitere Funktionen der Sprachanalysevorrichtung 10 in einem zentralen Server ausgeführt werden. So ist denkbar, auch den zweiten Computerlinguistik-Algorithmus P2 in einem Online-Modul zu hosten.

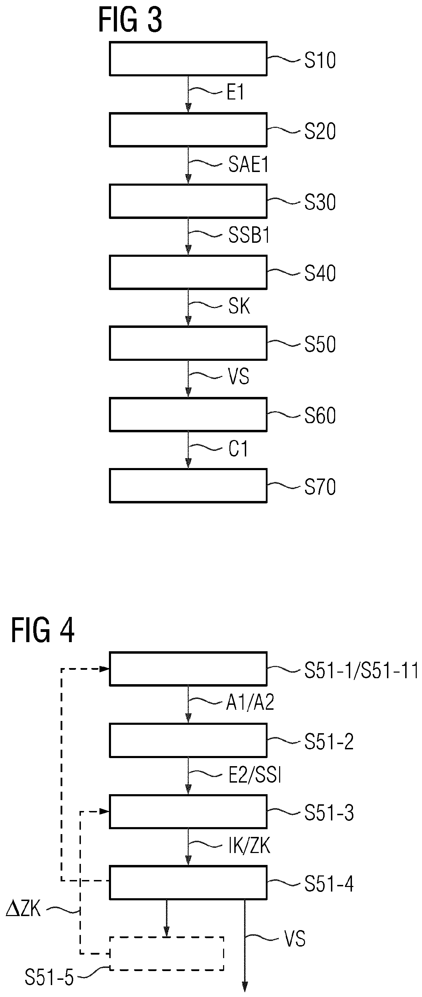

Eine Steuerung der medizinischen Vorrichtung 1 erfolgt in dem in Figur 1 beispielhaft dargestellten System 100 durch ein Verfahren, welches in **Figur 3** beispielhaft als Flussdiagramm dargestellt ist. Die Reihenfolge der Verfahrensschritte ist weder durch die dargestellte Abfolge noch durch die gewählte Nummerierung beschränkt. So kann die Reihenfolge der Schritte ggf. vertauscht werden. Einzelne Schritte können in Ausführungen parallel durchgeführt werden. Wiederum können einzelne Schritte weggelassen werden.

Allgemein ist hierbei vorgesehen, dass die Bedienperson, welche die medizinische Vorrichtung 1 bedient, ein Kommando stimmlich bzw. sprachlich äußert, z.B. indem sie einen Satz wie "Starte Scansequenz X" oder "bringe Patient in Ausgangsposition" spricht, die Eingabevorrichtung 2 ein zugehöriges Audiosignal E1 erfasst und verarbeitet und die Sprachsteuervorrichtung 10 das erfasste Audiosignal E1 analysiert und ein entsprechendes Steuerkommando C1 zur Betätigung der medizinischen Vorrichtung 1 erzeugt. Ein Vorteil dieses Vorgehens ist, dass die Bedienperson während des Sprechens auch andere Aufgaben erledigen kann, z.B. die Vorbereitung des Patienten. Dies beschleunigt vorteilhaft die Arbeitsabläufe. Ferner kann die medizinische Vorrichtung 1 dadurch zumindest teilweise "berührungslos" gesteuert werden, wodurch die Hygiene an der medizinische Vorrichtung 1 verbessert wird.

Das Verfahren zur Sprachsteuerung der medizinischen Vorrichtung 1 umfasst die Schritte S10 bis S70. Die Schritte werden bevorzugt mit der Sprachsteuervorrichtung 10 ausgeführt. In Schritt S10 erfolgt ein Erfassen eines Audiosignals E1 enthaltend eine auf die Steuerung der Vorrichtung 1 gerichtete Spracheingabe einer Bedienperson mittels der Eingabevorrichtung 2. Das Audiosignal E1 wird der Sprachsteuervorrichtung 10 über Eingang 31 bereitgestellt. Ein Schritt S20 umfasst ein Analysieren des Audiosignals E1 zum Bereitstellen eines ersten Sprachanalyseergebnisses SAE1. Schritt S20 umfasst also ein Bereitstellen einer zur Steuerung der medizinischen Vorrichtung relevanten sprachlichen Äußerung als erstes Sprachanalyseergebnis SAE1 aus dem Audiosignal E1. Das Erzeugen des Sprachanalyseergebnisses kann mehrere Teilschritte umfassen.

Ein Teilschritt kann darauf gerichtet sein, zunächst die im Audiosignal E1 enthaltene Schallinformation in Textinformation umzuwandeln, d.h. ein Transkript zu erzeugen. Ein Teilschritt kann darauf gerichtet sein, eine Tokenisierung des Audiosignals E1 bzw. der Spracheingabe der Bedienperson bzw. des Transkripts T vorzunehmen. Tokenisierung bezeichnet dabei die Segmentierung der Spracheingabe, d.h. den gesprochenen Text in Einheiten der Wort- bzw. Satzebene. Entsprechend kann das erste Sprachanalyseergebnis SAE1 eine erste Tokenisierungsinformation umfassen, welche beispielsweise angibt, ob die Bedienperson einen momentanen Satz zu Ende gesprochen hat oder nicht.

Ein Teilschritt kann darauf gerichtet sein, zusätzlich oder alternativ eine semantische Analyse des Audiosignals E1 bzw. der Spracheingabe der Bedienperson bzw. des Transkripts T auszuführen. Entsprechend kann das erste Sprachanalyseergebnis SAE1 eine erste semantische Information der Spracheingabe der Bedienperson umfassen. Die semantische Analyse ist dabei darauf gerichtet, der Spracheingabe eine Bedeutung zuzuordnen. Dazu kann beispielsweise Wortweise oder Wortgruppenweise ein Abgleich mit einer allgemeinen oder für die medizinische Vorrichtung 1 spezifischen Wort- und/oder Sprachbefehlsdatenbank 5 bzw. einer Wort- und/oder Sprachbefehlsbibliothek 50 der medizinischen Vorrichtung 1 bzw. des erfindungsgemäßen Systems 100 erfolgen. Insbesondere können in diesem Schritt bspw. dem Transkript T ein oder mehrere in einer Befehlsbibliothek 50 der medizinischen Vorrichtung 1 enthaltene Aussagen entsprechend verschiedener Sprachbefehle zuordenbar sind. So kann eine auf einen Sprachbefehl gerichtete Nutzerintention erkannt werden.

Die beschriebenen Teilschritte können von einem von Modul M1 umfassten Sprachverständnismodul ausgeführt werden, insbesondere können diese Teilschritte von dem Online-Modul OM1 ausgeführt werden.

In Schritt S30 erfolgt ein Erkennen eines ersten Sprachbefehls SSB1 basierend auf dem ersten Sprachanalyseergebnis SAE1. Hier wird insbesondere die semantische Information repräsentierend eine Nutzerintention genutzt, um einen entsprechenden Sprachbefehl als ersten Sprachbefehl SSB1 zuzuordnen. Anders ausgedrückt, wird der erkannte Sprachbefehl einer von vielen möglichen Befehlsklassen zugeordnet, wobei für eine Befehlsklasse verschiedene Ausdruckweisen, Worte, Wortfolgen oder Wortkombinationen hinterlegt sein können, die die dem Sprachbefehl entsprechende Nutzerintention repräsentieren können. Schritt S30 kann ebenfalls einen Abgleich des ersten Sprachanalyseergebnisses SAE1 mit einer Befehlsdatenbank 5 bzw. Befehlsbibliothek 50 umfassen.

In Schritt S40 erfolgt ein Zuordnen des ersten Sprachbefehls SSB1 zu einer Sicherheitsklasse SK. Hierbei ist mindestens eine Sicherheitsklasse für sicherheitskritische Sprachbefehle vorgesehen, zu welcher der erste Sprachbefehl SSB1 gehört. Dabei kann jeder Sprachbefehl mittels klassischer Lookup-Tabelle oder einer anderen Zuordnungsvorschrift einer Sicherheitsklasse SK zugeordnet sein. Erfindungsgemäß ist nun wenigstens eine der möglichen Sicherheitsklassen für sicherheitskritische Sprachbefehle vorgesehen, deren Ausführung durch die medizinische Vorrichtung 1 spezifischen, genormten Sicherheitsanforderungen genügen muss. Insbesondere sind hier Sprachbefehle umfasst, deren Ausführung erstfehlersicher realisiert werden muss.

Die Schritte S20 bis S40 können bspw. durch eine Software P1 realisiert werden, die auf dem Datenspeicher 4 abgelegt ist und die Recheneinheit 3, insbesondere Modul M1 oder Online-Modul OM1 zur Durchführung dieser Schritte veranlasst. Die Software P1 kann einen ersten Computerlinguistik-Algorithmus umfassend eine erste trainierte Funktion umfassen, die auf das Audiosignal, insbesondere zum Analysieren des Audiosignals E1, angewandt wird. Damit realisiert die Erfindung einen klassischen C-Pfad einer CP-Architektur.

Ist der erste Sprachbefehl SSB1 ein sicherheitskritischer Sprachbefehl, wird insbesondere Schritt S50 vollständig ausgeführt.

In Schritt S50 erfolgt ein Ermitteln eines Verifikationssignals VS zur Bestätigung des ersten Sprachbefehls SSB1. Das Verifikationssignal VS kann erfindungsgemäß verschieden ermittelt werden, wie mit Bezug zu den weiteren Figuren noch erläutert wird. Das Ermitteln des Verifikationssignals erfolgt erfindungsgemäß mit dem eigenständigen Modul M2 der Recheneinheit 3. Damit realisiert die Erfindung einen eigenständigen P-Pfad zur Absicherung sicherheitskritischer Sprachbefehle, wobei die Sicherheitsfunktion erfindungsgemäß auf einem mittels Methoden der maschinellen Sprachverarbeitung gewonnenen Sprachbefehls basiert.

In Schritt S60 erfolgt ein Erzeugen eines Steuersignals C1 zur Steuerung der medizinischen Vorrichtung basierend auf dem ersten Sprachbefehl SSB1 und dem Verifikationssignal VS, sofern der erste Sprachbefehl SSB1 in Schritt S50 bestätigt wurde, wobei das Steuersignal C1 geeignet ist, die medizinische Vorrichtung 1 entsprechend dem ersten Sprachbefehl SSB1 zu steuern. Mit anderen Worten wird der erste Sprachbefehl SSB1 in Form einer Eingangsvariable oder als Teil des Verifikationssignals dem Modul M3 der Recheneinheit 3 (oder einer entsprechenden Software, die beispielsweise auf dem Datenspeicher 4 abgelegt ist) übergeben und wenigstens ein Steuersignal C1 daraus abgeleitet. In einem Schritt S70 erfolgt die Eingabe bzw. Übergabe des Steuersignals C1 in die medizinische Vorrichtung 1 über den Ausgang 32.

**Figur** 4 zeigt ein Ablaufdiagramm zur Ermittlung eines Verifikationssignals VS für den ersten Sprachbefehl SSB1 in einer Ausführung der Erfindung, in der die Bestätigung des ersten Sprachbefehls SSB1 eine Nutzerinteraktion erfordert. Die Nutzerinteraktion erfolgt bevorzugt wieder per Sprache, sodass auch in dieser Ausführung eine einfache und schnelle Bedienung der medizinischen Vorrichtung 1 sowie ein hoher Hygiene-Standard gewahrt sind.

Die in Figur 4 dargestellten Schritte, deren Reihenfolge durch den Ablauf ebenfalls nicht zwingend vorgegeben ist, können im Rahmen von Schritt S50 aus Figur 3 erfolgen. Dementsprechend umfasst das Ermitteln des Verifikationssignals VS einen Schritt S51-1, in dem der erste Sprachbefehl SSB1 sowie eine Aufforderung A1 zur Bestätigung des ersten Sprachbefehls SSB1 an die Bedienperson ausgegeben wird. In bevorzugter Ausführung erfolgt die Ausgabe der Aufforderung A1 und des ersten Sprachbefehls SSB1 in Form eines Audiosignals. In anderen Ausführungen kann die Ausgabe allerdings auch anders, bspw. mittels einer optischen Ausgabeschnittstelle in Form eines Displays erfolgen. Die Aufforderung A1 kann eine Information umfassen, dass der erste Sprachbefehl SSB1 erkannt wurde und welche Nutzereingabe (Inhaltskriterium IK) konkret zu dessen Bestätigung erforderlich ist und/oder innerhalb welcher Zeitspanne (Zeitkriterium) die Bestätigung erfolgen muss. Bspw. kann die Aufforderung lauten: '*Kommando XY erkannt, bitte binnen 5 sec mit 'JA' bestätigen'*. Das Modul M2 ist bspw. ausgebildet, basierend auf dem ersten Sprachbefehl SSB1 einen strukturierten Sprachdatenstrom und darauf basierend eine Schallinformation bereitzustellen, die dort erzeugt und der Ausgabevorrichtung 35 des Ausgangs 32 zur Umwandlung in ein Schallsignal zugeführt wird. Dazu kann eine Software Pn im Speicher 4 durch Modul M2 abrufbar abgelegt sein. Die Software Pn kann einen weiteren Computerlinguistik-Algorithmus Pn zur maschinellen Erzeugung natürlicher Sprache umfassen. Der weitere Computerlinguistik-Algorithmus Pn kann hier als Algorithmus der natürlichen Spracherzeugung (NLG) ausgebildet sein. Alternativ kann für eine Vielzahl von Befehlsklassen, in einer entsprechenden Ausgabedatenbank jeweils ein vorab definierter strukturierter Sprachdatenstrom oder ein Schallsignal abrufbar hinterlegt sein, der von Modul M2 entsprechend dem ersten Sprachbefehl SSB1 erkannt und an den Ausgang zur Erzeugung des Audiosignals A1 übergeben wird.

In einem optionalen Schritt S51-11 kann die Aufforderung zur Benutzereingabe A1 um eine optionale Abfrage A2 einer für den ersten Sprachbefehl SSB1 spezifischen Sprachsteuerinformation SSI bereichert werden, die ebenfalls wie das Audiosignal A1 an die Bedienperson ausgegeben wird. Unter einer Sprachsteuerinformation SSI ist dabei eine für die Ausführung des ersten Sprachbefehls SSB1 notwendige Information, die spezifische, notwendige Parameter oder Angaben, bspw. eine Längenangabe entsprechend einem gewünschten Verstellweg, umfassen kann. Die optionale Abfrage A2 kann bspw. lauten: '*Um welche Wegstrecke (in cm) soll die Liege nach oben verfahren werden?'*.

In Schritt S51-2 erfolgt nun ein Erfassen einer auf die Bestätigung des ersten Sprachbefehls gerichteten Benutzereingabe E2 der Bedienperson, hier in Form eines Audiosignals E2. Das Erfassen kann, wie oben bereits mit Bezug zu Audiosignal E1 beschrieben, über den Eingang 31 der Sprachsteuervorrichtung 10 und die Eingabevorrichtung 2 erfolgen und entsprechende Vorverarbeitungsschritte (Digitalisierung, Speicherung, etc.) umfassen. Optional kann über den Eingang 31 und die Eingabevorrichtung auch eine die abgefragte Sprachsteuerinformation SSI umfassende Benutzereingabe miterfasst und dem Modul M2 zur Weiterverarbeitung zugeführt werden. Entsprechend kann das Audiosignal E2 dabei auch die Sprachsteuerinformation SSI umfassen.

Schritt S51-3 ist auf ein Auswerten des Audiosignals E2 durch Modul M2 gerichtet, um den Inhalt des Audiosignals zu erschließen. Dazu kann eine Software, die ebenfalls im Speicher 4 abrufbar hinterlegt sein kann, insbesondere in Form eines weiteren Computerlinguistik-Algorithmus Pn vorgesehen sein. Dieser weitere Computerlinguistik-Algorithmus Pn kann in Ausführungen ausgebildet sein, insbesondere kurze, auf eine Befehlsbestätigung gerichtete Schlüsselworte wie 'Ja', 'Yes', 'Yes. Please. ' ‚Confirmed', ‚Check' oder dergleichen zu erkennen.

Daneben kann der weitere Computerlinguistik-Algorithmus Pn auch ausgebildet sein, eine Sprachsteuerinformation SSI aus dem Audiosignal E2 abzuleiten. Für jede Befehlsklasse bzw. für jeden Sprachbefehl können individuell festgelegte Schlüsselworte definiert und hinterlegt sein, die der weitere Computerlinguistik-Algorithmus Pn in dem Audiosignal E2 erkennen muss.

Ein Teilschritt kann auch hier auf eine Tokenisierung des zweiten Audiosignals E2 und das Bereitstellen einer Tokenisierungsinformation gerichtet sein. Ein weiterer Teilschritt kann auch hier auf eine semantische Analyse des zweiten Audiosignals E2 und das Bereitstellen einer semantischen Information gerichtet sein, wie mit Bezug zum ersten Computerlinguistik-Algorithmus beschrieben.

Modul M2 überprüft in Schritt S51-3 also, ob im Audiosignal umfasste Worte oder Wortfolgen einem für den ersten Sprachbefehl SSB1 hinterlegten Schlüsselwort entsprechen. Modul M2 prüft damit, ob ein vorab definiertes Inhaltskriterium IK der auf die Bestätigung gerichteten Benutzereingabe E2 erfüllt ist oder nicht. Nur bei Erkennen des hinterlegten Schlüsselwortes ist das Inhaltskriterium IK erfüllt.

Alternativ oder zusätzlich prüft Modul M2 auch, ob die Benutzereingabe ein Zeitkriterium ZK erfüllt. Dazu kann ein für den ersten Sprachbefehl SSB1 spezifischer und hinterlegter zeitlicher Schwellwert entsprechend einer maximalen Zeitspanne oder Dauer überwacht werden, in welchem das Audiosignal E2 nach Ausgabe der Aufforderung A1 erfasst werden muss. Nur wenn die Eingabe schnell genug erfolgt, ist das Zeitkriterium ZK erfüllt.

Ist das Inhaltskriterium IK und/oder das Zeitkriterium ZK erfüllt, ist der erste Sprachbefehl SSB1 verifiziert bzw. bestätigt.

In Ausführungen der Erfindung können insbesondere die Schritte S51-1 bis S51-3 mehrfach, bspw. zwei oder drei Mal durchlaufen werden. Diese mehreren Verifikationsschleifen können insbesondere für sicherheitskritische Sprachbefehle mit hoher Sicherheitsstufe, insbesondere der Sicherheitsklasse umfassend erstfehlersichere Sprachbefehle, vorgesehen sein.

Modul M2 der Sprachsteuervorrichtung 10 erzeugt in Schritt S51-4 weiter ein Verifikationssignal VS basierend auf dem Prüfergebnis aus Schritt S51-3. Das Prüfergebnis kann dabei die Ergebnisse aller durchlaufenen Prüfschleifen oder nur das Ergebnis der letzten durchlaufenen Prüfschleife berücksichtigen. Das Verifikationssignal VS wird im Folgenden an Modul M3 entsprechend einer Steuereinheit der Sprachsteuervorrichtung übertragen, welche (Schritt S60, Fig. 3) das Steuersignal C1 entsprechend dem ersten, mittels Verifikationssignal VS bestätigten Sprachbefehl SSB1 generiert.

Sicherheitsanforderungen an die Ausführung eines sicherheitskritischen Sprachbefehls werden in dieser Ausführung der Erfindung eingehalten, indem basierend auf dem ersten erkannten Sprachbefehl SSB1 eine Sicherheitsfunktion in Form der durch Modul M2 ausgeführten Verifikationsschleife(n) gestartet wird, bei der die Verifikation über eine manuelle Bestätigung per Nutzereingabe, also klassisch implementiert ist. Durch die Überwachung des Inhaltskriteriums IK und des Zeitkriteriums ZK ist die implementierte Sicherheitsfunktion konventionell nachweisbar und sicherheitstechnisch belastbar, obwohl das Ausgangssignal für die Verifikationsschleife (der erste Sprachbefehl SSB1) aus einer sicherheitstechnisch nicht belastbaren Analyse stammt. Sofern also Modul M1 bei der Identifikation des ersten Sprachbefehls SSB1 einen Fehler macht, wird dieser durch Modul M2 korrigiert und der erste Sprachbefehl SSB1 wird nicht ausgeführt.

Die Variabilität des Eingaberaumes, also die Möglichkeit zur Begrenzung bzw. Erweiterung der zulässigen Kommandos/Befehle für den ersten oder den weiteren Computerlinguistik-Algorithmus, welche auf die Audiosignale E1, E2 angewandt werden, erleichtert zudem die Verifikation des erfindungsgemäßen Systems und ermöglicht eine Vereinfachung der anzunehmenden Fehlermodelle. Das erfindungsgemäße System wird ferner in einer an sich bekannten Umgebung eingesetzt, daher sind auch mögliche Störeinflüsse wie bspw. Hintergrundgeräusche weitgehend bekannt. Das erleichtert die Erstellung von Robustheitstest für das erfindungsgemäße System sowie das Erzeugen von kritischen, zu erwartenden Eingabeszenarien.

In einem weiteren optionalen Schritt S51-5 kann ein Anpassen des initial vorgegebenen Zeitkriteriums ZK in Abhängigkeit des ersten Sprachbefehls SSB1 erfolgen. Insbesondere Schritt S51-1 kann zeitlich losgelöst von den übrigen Schritten S51-1 bis S51-4 durchgeführt werden. Das Anpassen des Zeitkriteriums ZK umfasst in Ausführungen das Addieren oder Subtrahieren einer Zeitdifferenz $\Delta$ZK zu der bisherigen als Schwellwert definierten Zeitspanne. Die Anpassbarkeit des Zeitkriteriums ZK dient vor allem einer Skalierbarkeit des erfindungsgemäßen Verfahrens hin zu einer höheren Bedienfreundlichkeit ($\Delta$ZK wird aufaddiert) bzw. zu einer höheren Befehlssicherheit ($\Delta$ZK wird subtrahiert).

In weiteren Ausführungen (nicht dargestellt) kann im Sinne einer flexiblen Skalierbarkeit des Verfahrens ferner ein Schritt vorgesehen sein, alternativ oder zusätzlich das Inhaltskriterium anzupassen IK, sodass bspw. mehr oder weniger Schlüsselworte als zu einem Sprachbefehl dazugehörig erkannt werden können.

Auch die Anzahl der Verifikationsschleifen, die vor Erstellung des Verifikationssignals durchlaufen werden müssen, können im Sinne einer Skalierbarkeit verändert werden.

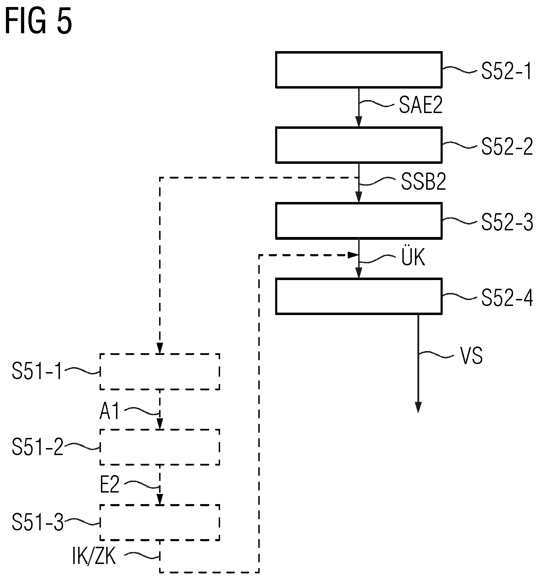

**Figur 5** zeigt ein weiteres Ablaufdiagramm zur Ermittlung eines Verifikationssignals VS für den ersten Sprachbefehl SSB1 in einer weiteren Ausführung der Erfindung, in der eine Bestätigung des ersten Sprachbefehls SSB1 ohne Nutzerinteraktion erfolgen kann. Mit anderen Worten ermöglicht die Erfindung in dieser Ausführung eine besonders einfache und schnelle Bedienung der medizinischen Vorrichtung 1 mit nur einer initialen Spracheingabe im Sinne des Audiosignals E1.

Die in Figur 5 dargestellten Schritte, deren Reihenfolge durch den Ablauf ebenfalls nicht zwingend vorgegeben ist, können genauso im Rahmen von Schritt S50 aus Figur 3 erfolgen. Dementsprechend umfasst Schritt S50, also das Ermitteln des Verifikationssignals VS, die folgenden Schritte.

Schritt S52-1 ist auf ein Analysieren des Audiosignals E1 zum Bereitstellen eines zweiten Sprachanalyseergebnisses SAE2 gerichtet und Schritt S52-2 ist auf ein Erkennen eines zweiten Sprachbefehls SSB2 basierend auf dem zweiten Sprachanalyseergebnis SAE2.

Die Schritte S52-1 und S52-2 werden durch eine Software P2 realisiert werden, die auf dem Datenspeicher 4 abgelegt ist und die Recheneinheit 3, insbesondere Modul M2 zur Durchführung dieser Schritte veranlasst. Die Software P2 kann einen zweiten Computerlinguistik-Algorithmus P2 umfassend eine zweite trainierte Funktion umfassen, die auch, so wie Software P1 auf das Audiosignal E1, insbesondere zum Analysieren des Audiosignals E1, angewandt wird.

Schritt S52-1 kann ebenfalls eine Tokenisierung des Audiosignals E1 und/oder eine semantische Analyse des Audiosignals E1 umfassen.

Der zweite Computerlinguistik-Algorithmus P2 kann vorteilhaft eine zweite trainierte Funktion umfassen. Kennzeichnend für die vorliegende Erfindung ist, dass sich die erste trainierte Funktion und die zweite trainierte Funktion voneinander unterscheiden. Insbesondere ist die zweite trainierte Funktion ausgebildet, nur sicherheitskritische, insbesondere erstfehlersicher abzubildende Sprachbefehle in dem Audiosignal E1 zu identifizieren, wohingegen die erste trainierte Funktion ausgebildet ist, einen sehr breiten Wortschatz bzgl. verschiedenster Spracheingaben einer Bedienperson und insbesondere auch nicht sicherheitskritische Sprachbefehle zu erkennen. Die zweite trainierte Funktion ist in diesem Sinne spezifisch für sicherheitskritische Sprachbefehle ausgebildet. Die zweite trainierte Funktion erkennt also keine nicht sicherheitskritischen Sprachbefehle bzw. allg. Spracheingaben. Insofern ist zumindest Schritt S52-2 spezifisch für das Erkennen eines sicherheitskritischen Sprachbefehls als zweiten Sprachbefehl SSB2.

Sicherheitskritische Sprachbefehle sind durch eine charakteristische Merkmalskombination, aus bspw. dem Frequenzmuster, der Amplitude, der Modulation, oder dergleichen gekennzeichnet. Sicherheitskritische, insbesondere erstfehlersichere Sprachbefehle weisen zudem insbesondere eine Mindest-Silbenanzahl von drei oder mehr auf. Je größer die Anzahl der Silben eines Sprachbefehls, umso größer einfacher kann er von anderen Sprachbefehlen unterschieden werden. Eine Verwechslungsgefahr zu anderen Sprachbefehlen wird dadurch minimiert.

Die Spezifität der Schritte S52-1 und S52-2 wird erreicht, indem die zweite trainierte Funktion anders als die erste trainierte Funktion ausgebildet wird. Ein wesentlicher Unterschied zwischen der ersten und der zweiten trainierten Funktion kann zwischen den jeweils verwendeten Trainingsdaten liegen. Der erste Trainingsdatensatz für die erste trainierte Funktion umfasst einen breiten und vielseitigen Sprachschatz bzgl. verschiedenster Sprachbefehle bzw. allgemeiner Spracheingaben. Der zweite Trainingsdatensatz für die zweite trainierte Funktion ist hingegen auf einen Sprachschatz beschränkt, der auf spezifische, insbesondere phonetisch eindeutige Sprachbefehle gerichtet ist, um sicherheitskritische, insbesondere erstfehlersichere Sprachbefehle eindeutig mit geringer Fehlerrate erkennen zu können. Insofern kann die zweite trainierte Funktion erfindungsgemäß mittels eines kleinen Trainings-Wortschatz (engl. small vocabulary) und im Gegensatz dazu die erste trainierte Funktion mit einem großen Trainings-Wortschatz (engl. large vocabulary) trainiert werden. Auch die trainierten Funktionen selbst können sich erfindungsgemäß unterscheiden, speziell in der der Ausbildung der Verifikationsfunktion bzw. der Klassifizierungsfunktion. Die erste trainierte Funktion weist eine große Anzahl Kategorien entsprechend einer Vielzahl verschiedener Sprachbefehle auf. Die zweite trainierte Funktion hingegen ist auf eine geringere Menge Kategorien entsprechend einer kleinen, insbesondere für die medizinische Vorrichtung spezifischer sicherheitskritischer Sprachbefehle beschränkt. Durch den Einsatz unterschiedlicher trainierter Funktionstypen reduziert die Erfindung das Risiko für das Auftreten ähnlicher systematischer Fehler bei der Sprachbefehlserkennung durch die erste trainierte Funktion und die zweite trainierte Funktion.

Identifiziert die zweite trainierte Funktion in Schritt S52-2 einen Sprachbefehl als zweiten Sprachbefehl SSB2 aus dem zweiten Sprachanalyseergebnis SAE2, handelt es sich also per se um einen sicherheitskritischen Sprachbefehl.

Durch die verschiedenartige Ausbildung der ersten und zweiten trainierten Funktionen wird in dieser Ausführung das Risiko einer Ausführung eines fehlerhaft im Modul M1 erkannten ersten Sprachbefehls SSB1 minimiert, da systematische Sprachbefehlserkennungsfehler der ersten trainierten Funktion mit großer Wahrscheinlichkeit nicht von der zweiten trainierten Funktion wiederholt werden.

In einem Schritt S52-3 wird nun die Übereinstimmung zwischen dem ersten und dem zweiten Sprachbefehl SSB1, SSB2 anhand eines Übereinstimmungskriteriums ÜK geprüft. Dabei wird ein Übereinstimmungsmaß zwischen den beiden Sprachbefehlen mit einem für den ersten und/oder zweiten Sprachbefehl SSB1/SSB2 spezifischen und vorab festgelegten Schwellwert verglichen. Der Schwellwert bzw. das erforderliche Ähnlichkeitsmaß kann von Sprachbefehl zu Sprachbefehl unterschiedlich groß ausfallen. Schwellwerte von Sprachbefehlen einer hohen Sicherheitsstufe, also insbesondere erstfehlersicher abzubildenden Sprachbefehlen, sind erfindungsgemäß größer ausgebildet als von Sprachbefehlen einer niedrigeren Sicherheitsstufe.

Ergibt der Prüfschritt S52-3, dass der erste und der zweite Sprachbefehl SSB1, SSB2 ein bei oder oberhalb des Schwellwertes liegendes Übereinstimmungsmaß aufweisen, ist der erste Sprachbefehl SSB1 bestätigt.

Modul M2 der Sprachsteuervorrichtung 10 erzeugt in Schritt S52-4 weiter ein Verifikationssignal VS basierend auf dem Prüfergebnis aus Schritt S53-3. Das Verifikationssignal VS wird im Folgenden an Modul M3 entsprechend der Steuereinheit der Sprachsteuervorrichtung 10 übertragen, welche (in Schritt S60 in Fig. 3) das Steuersignal C1 entsprechend dem ersten, mittels Verifikationssignal VS bestätigten Sprachbefehl SSB1 generiert.

Optional können dem in Figur 5 dargestellten Verfahren die Schritte S51-1 bis S51-3 hinzu ergänzt werden, nachdem in Schritt S52-2 der zweite, sicherheitskritische Sprachbefehl SSB2 erkannt wurde, wobei das Prüfergebnis von Schritt S51-3 ebenfalls in die Erzeugung des Verifikationssignals in Schritt S52-4 eingeht. Anders ausgedrückt hängt in dieser Ausführung das Verifikationssignal VS auch vom Prüfergebnis des Schritts S51-3 ab.

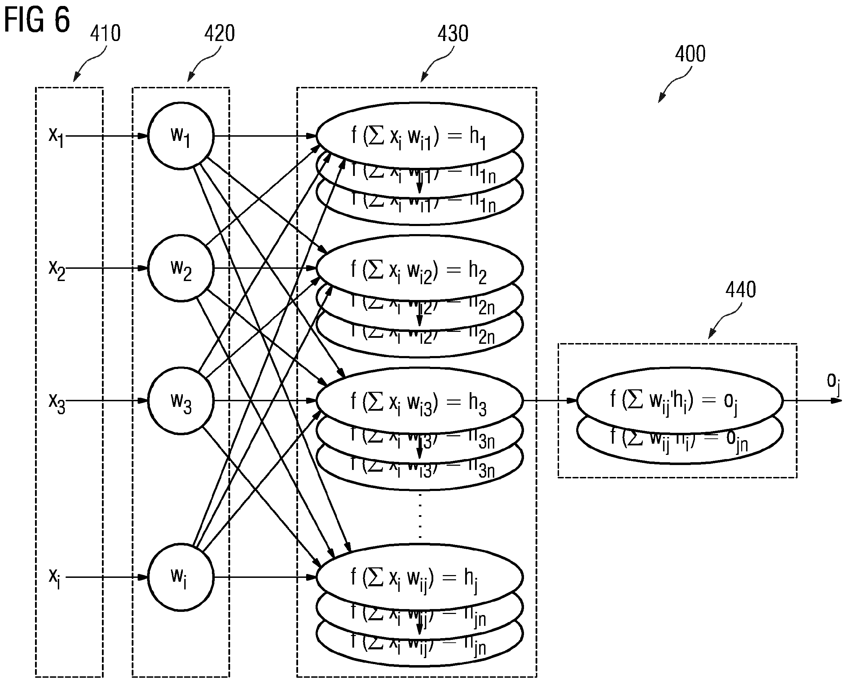

**Figur 6** zeigt ein künstliches neuronales Netz 400, wie es in Verfahren gemäß der Figuren 3 bis 5 zum Einsatz kommen kann. Insbesondere kann es sich bei dem gezeigten neuronalen Netz 400 um die erste oder zweite trainierte Funktion des ersten bzw. des zweiten Computerlinguistik-Algorithmus P1, P2 handeln. Das neuronale Netz 400 antwortet auf Eingabewerte zu einer Vielzahl von Eingangsknoten $x_i$ 410 die angewendet werden, um eine oder eine Vielzahl von Ausgaben $o_j$ zu erzeugen. Das neuronale Netz 400 lernt in diesem Ausführungsbeispiel, indem es die Gewichtungsfaktoren $w_i$ (weights) der einzelnen Knoten basierend auf Trainingsdaten anpasst. Mögliche Eingabewerte der Eingangsknoten $x_i$ 410 können bspw. Spracheingaben bzw. Audiosignale eines ersten oder eines zweiten Trainingsdatensatzes sein. Das neuronale Netz 400 gewichtet 420 die Eingabewerte 410 basierend auf dem Lernprozess. Die Ausgabewerte 440 des neuronalen Netzes 400 entsprechen in Ausführungen einem ersten bzw. einem zweiten Sprachbefehl SSB1, SSB2. Die Ausgabewerte 440 des neuronalen Netzes können in anderen Ausführungen auch eine Angabe zu einer Sicherheitsklasse SK des ersten Sprachbefehls SSB1 oder ein Ergebnis einer Überprüfung eines Übereinstimmungskriteriums zwischen erstem und zweitem Sprachbefehl SSB1, SSB2 umfassen. Die Ausgabe 440 kann über einen einzelnen oder eine Vielzahl von Ausgabeknoten $o_j$ erfolgen.

Das künstliche neuronale Netz 400 umfasst bevorzugt eine versteckte Schicht 430, die eine Vielzahl von Knoten $h_j$ umfasst. Es können mehrere versteckte Schichten $h_{jn}$ vorgesehen sein, wobei eine versteckte Schicht 430 Ausgabewerte einer anderen (versteckten) Schicht 430 als Eingabewerte verwendet. Die Knoten einer versteckten Schicht 430 verrichten mathematische Operationen. Ein Ausgabewert eines Knotens $h_j$ entspricht dabei einer nicht-linearen Funktion f seiner Eingabewerte $x_i$ und der Gewichtungsfaktoren $w_i$. Nach dem Erhalt von Eingabewerten $x_i$ führt ein Knoten $h_j$ eine Summierung einer mit den Gewichtungsfaktoren $w_i$ gewichteten Multiplikation jedes Eingabewerts $x_i$ durch, wie durch folgende Funktion bestimmt:

$$h_j = f\left(\sum_i x_i \cdot w_{ij}\right)$$

Insbesondere wird ein Ausgabewert eines Knotens $h_j$ als Funktion f einer Knoten-Aktivierung, bspw. eine Sigmoidalfunktion oder eine lineare Rampenfunktion gebildet. Die Ausgabewerte $h_j$ werden an den bzw. die Ausgabeknoten $o_j$ übertragen. Erneut wird eine Summierung einer gewichteten Multiplikation jedes Ausgabewertes $h_j$ als Funktion der Knoten-Aktivierung f berechnet:

$$o_j = f\left(\sum_i h_i \cdot w'_{ij}\right)$$

Das hier gezeigte neuronale Netz 400 ist ein vorwärts gerichtetes (engl. feedforward) neuronales Netz, wie es bevorzugt für den ersten Computerlinguistik-Algorithmus P1 zum Einsatz kommt, bei dem alle Knoten 430 die Ausgabewerte einer vorherigen Schicht in Form ihrer gewichteten Summe als Eingabewerte verarbeiten. Insbesondere für den zweiten Computerlinguistik-Algorithmus können erfindungsgemäß andere neuronale Netztypen zum Einsatz kommen, bspw. rückkoppelndes, bspw. selbstlernendes (engl. recurrent) neuronales Netz, bei denen ein Ausgabewert eines Knotens $h_j$ gleichzeitig auch sein eigener Eingabewert sein kann.

Das neuronale Netz 400 wird bevorzugt mittels einer Methode des überwachten Lernens trainiert, um Muster zu erkennen. Eine bekannte Vorgehensweise ist die Back-Propagation, die für alle Ausführungsbeispiele der Erfindung angewandt werden kann. Während des Trainings wird das neuronale Netz 400 auf Trainings-Eingabewerten angewandt und muss entsprechende, vorher bekannte Ausgabewerte erzeugen. Iterativ werden mittlere quadratische Fehler (mean square error - "MSE") zwischen berechneten und erwarteten Ausgabewerten berechnet und einzelne Gewichtungsfaktoren 420 so lange angepasst, bis die Abweichung zwischen berechneten und erwarteten Ausgabewerten unterhalb einer vorbestimmten Schwelle liegt.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

Die Erfindung ermöglicht die Nutzung von Computerlinguistik-Algorithmen umfassend neuronale Netze zur Spracherkennung und Ableitung von Sprachbefehlen in sicherheitsrelevanten Anwendungen, die insbesondere erstfehlersicher ausgebildet sein müssen. In Tests konnte belegt werden, dass die beschriebene Lösung zuverlässiger als klassisch implementierte Spracherkennungsalgorithmen ohne Sicherheitsfunktion sind. Die Erfindung ist in Ausführungen durch eine Skalierbarkeit hinsichtlich Benutzerfreundlichkeit und/oder Sicherheit (Fehleraufdeckung) gekennzeichnet. Dies kann einerseits genutzt werden, um Sprachbefehle unterschiedlicher Sicherheitsstufen jeweils angemessenen abzusichern. Andererseits ist es möglich, mit der Zeit die Bedienbarkeit zu verbessern, wenn Erfahrungen vorliegen, dass eine Sprachbefehlserkennung bzw. Sprachbefehlsverifikation in einer definierten Zielumgebung gewünschten Sicherheitsvorgaben entspricht und eine ausreichende Zuverlässigkeit nachgewiesen werden konnte. Alternativ ist es möglich, die Sicherheit zu erhöhen, wenn erkannt wurde, dass bspw. aufgrund spezieller Umgebungsbedingungen (bspw. Hintergrundgeräusche) eine zuverlässige Erkennung der per Spracheingabe übermittelten Kommandos nicht ausreichend gut funktioniert.

Die Skalierbarkeit ermöglicht allgemein eine graduelle Änderung der sicherheitstechnischen Belastung zwischen konventionell implementierten Anteilen und über Methoden des Maschinellen Lernens implementierten Anteilen. Hierdurch kann ein Migrationspfad in Richtung der sicherheitstechnisch belastbaren Nutzung von Computerlinguistik-Algorithmen umfassend neuronale Netze geschaffen werden.

Abschließend sei darauf hingewiesen, dass die erfindungsgemäße Ausbildung eines Verifikationsmechanismus für mittels Spracherkennung abgeleitete Steuerbefehle in Bezug auf eine falsche Interpretation erkannter Kommandos (einer eine Nutzerintention umfassende Spracheingabe wird ein falscher Sprachbefehl zugeordnet) oder in Bezug auf eine unzeitige, also zu langsame Erkennung von Sprachkommandos angewendet werden kann. Erfindungsgemäß kann jedoch ein Fehler, bei dem mit dem ersten Computerlinguistik-Algorithmus in einer Spracheingabe trotz enthaltener Nutzerintention kein Sprachbefehl erkannt wird, nicht abgesichert werden. Daher ist die vorliegende Erfindung nicht geeignet, bspw. eine Not-Stopp Funktion abzusichern.

**Patentansprüche**

1.  Verfahren zur Sprachsteuerung einer medizinischen Vorrichtung (1) mit den Schritten:

    - Erfassen (S10) eines Audiosignals (E1) enthaltend eine auf die Steuerung der Vorrichtung gerichtete Spracheingabe einer Bedienperson;
    - erstes Analysieren (S20) des Audiosignals zum Bereitstellen eines ersten Sprachanalyseergebnisses (SAE1),
    - Erkennen (S30) eines ersten Sprachbefehls (SSB1) basierend auf dem ersten Sprachanalyseergebnis,
    - Zuordnen (S40) des ersten Sprachbefehls zu einer Sicherheitsklasse (SK), wobei eine Sicherheitsklasse für sicherheitskritische Sprachbefehle vorgesehen ist,
    - Ermitteln (S50) eines Verifikationssignals (VS) zur Bestätigung des ersten Sprachbefehls, wenn der erste Sprachbefehl einer Sicherheitsklasse für sicherheitskritische Sprachbefehle zugeordnet wurde,
    - Erzeugen (S60) eines Steuersignals (C1) zur Steuerung der medizinischen Vorrichtung basierend auf dem ersten Sprachbefehl und dem Verifikationssignal, sofern der erste Sprachbefehl bestätigt wurde, wobei das Steuersignal geeignet ist, die medizinische Vorrichtung entsprechend dem ersten Sprachbefehl zu steuern, und
    - Eingabe (S70) des Steuersignals in die medizinische Vorrichtung
    wobei
    - das Ermitteln des Verifikationssignals ein Ausgeben des ersten Sprachbefehls und einer Aufforderung (A1) zur Bestätigung des ersten Sprachbefehls an die Bedienperson (S51-1) sowie ein Erfassen (S51-2) einer auf die Bestätigung des ersten Sprachbefehls gerichteten Benutzereingabe (E2) der Bedienperson umfasst,
    - das Verifikationssignal den ersten Sprachbefehl bestätigt, wenn die auf die Bestätigung des ersten Sprachbefehls gerichtete Benutzereingabe der Bedienperson
    - ein vorab definiertes Zeitkriterium (ZK) erfüllt, und
    - das Zeitkriterium in Abhängigkeit des ersten Sprachbefehls nach Benutzer-spezifischen Vorgaben anpassbar ist.

2.  Verfahren nach Anspruch 1, wobei

    - das Ausgeben ein Ausgeben eines auf dem Sprachbefehl basierenden Audiosignals (A1), und/oder
    - das Erfassen ein Erfassen einer als Audiosignal ausgebildeten Benutzereingabe (E2) umfasst.

3.  Verfahren nach einem der Ansprüche 1 bis 2, wobei das Verifikationssignal den ersten Sprachbefehl bestätigt, wenn die auf die Bestätigung des ersten Sprachbefehls gerichtete Benutzereingabe der Bedienperson

    - ein vorab definiertes Inhaltskriterium (IK) erfüllt.

4.  Verfahren nach einem der Ansprüche 1 bis 3, wobei das Ermitteln des Verifikationssignals auch umfasst, eine Aufforderung (A2) zur Benutzereingabe einer für den ersten Sprachbefehl spezifischen Sprachsteuerinformation (SSI) an die Bedienperson auszugeben, und eine die Sprachsteuerinformation umfassende Benutzereingabe zu erfassen.

5.  Verfahren nach einem der vorherigen Ansprüche, wobei das Analysieren ein Anwenden eines ersten Computerlinguistik-Algorithmus umfassend eine erste trainierte Funktion auf das Audiosignal umfasst.

6.  Verfahren nach einem der vorherigen Ansprüche, wobei das Ermitteln des Verifikationssignals umfasst

    - Analysieren (S52-1) des Audiosignals zum Bereitstellen eines zweiten Sprachanalyseergebnisses (SAE2),
    - Erkennen (S52-3) eines zweiten Sprachbefehls (SSB2) basierend auf dem zweiten Sprachanalyseergebnis,
    - Vergleichen (S52-4) des ersten und des zweiten Sprachbefehls,

    wobei das Verifikationssignal den ersten Sprachbefehl bestätigt, wenn der erste Sprachbefehl und der zweite Sprachbefehl ein Übereinstimmungskriterium (ÜK) erfüllen.

7.  Verfahren nach Anspruch 5 und 6, wobei das Analysieren ein Anwenden eines zweiten Computerlinguistik-Algorithmus umfassend eine zweite trainierte Funktion auf das Audiosignal umfasst, wobei sich die erste trainierte Funktion und die zweite trainierte Funktion voneinander unterscheiden.

8.  Verfahren nach Anspruch 7, wobei die zweite trainierte Funktion ausgebildet ist, nur sicherheitskritische Sprachbefehle in dem Audiosignal zu identifizieren.

**9.** Verfahren nach einem der vorherigen Ansprüche, wobei das Analysieren des Audiosignals

- ein Tokenisieren zur Segmentierung von Buchstaben, Wörtern und/oder Sätzen innerhalb des Audiosignals umfassen und der erste und/oder der zweite Sprachbefehl basierend auf einer ersten Tokenisierungsinformation und/oder einer zweiten Tokenisierungsinformation erkannt werden,
und/oder
- eine semantische Analyse des Audiosignals umfassen und der erste und der zweite Sprachbefehl basierend auf einer ersten semantischen Information und einer zweiten semantischen Information erkannt werden.

**10.** Sprachsteuervorrichtung (10) zur Sprachsteuerung einer medizinischen Vorrichtung (1), umfassend

- wenigstens eine Schnittstelle (31, 34) zum Erfassen eines Audiosignals (E1) enthaltend eine auf die Steuerung der Vorrichtung gerichtete Spracheingabe einer Bedienperson;
- wenigstens eine Auswerteeinheit (3, M1, M2, OM1), die ausgebildet ist,

- das Audiosignal zu analysieren und ein erstes Sprachanalyseergebnis (SAE1) bereitzustellen,
- einen ersten Sprachbefehl (SSB1) basierend auf dem ersten Sprachanalyseergebnis zu erkennen,
- den ersten Sprachbefehl zu einer Sicherheitsklasse (SK) zuzuordnen, wobei eine Sicherheitsklasse für sicherheitskritische Sprachbefehle vorgesehen ist,
- ein Verifikationssignal (VS) zur Bestätigung des ersten Sprachbefehls zu ermitteln, wenn der erste Sprachbefehl einer Sicherheitsklasse für sicherheitskritische Sprachbefehle zugeordnet wurde,
wobei

- die Auswerteeinheit ausgebildet ist, den ersten Sprachbefehl und eine Aufforderung (A1) zur Bestätigung des ersten Sprachbefehls an die Bedienperson auszugeben (S51-1) und eine auf die Bestätigung des ersten Sprachbefehls gerichtete Benutzereingabe (E2) der Bedienperson zu erfassen,
- das Verifikationssignal den ersten Sprachbefehl bestätigt, wenn die auf die Bestätigung des ersten Sprachbefehls gerichtete Benutzereingabe der Bedienperson
- ein vorab definiertes Zeitkriterium (ZK) erfüllt, und
- das Zeitkriterium in Abhängigkeit des ersten Sprachbefehls nach Benutzerspezifischen Vorgaben anpassbar ist, und

- eine Steuereinheit (3, M3), die ausgebildet ist,

- ein Steuersignal (C1) zur Steuerung der medizinischen Vorrichtung basierend auf dem ersten Sprachbefehl und dem Verifikationssignal zu erzeugen, sofern der erste Sprachbefehl anhand des Verifikationssignals bestätigt wurde, wobei das Steuersignal geeignet ist, die medizinische Vorrichtung entsprechend dem ersten Sprachbefehl zu steuern, und

- eine Schnittstelle (32) zur Eingabe des Steuersignals in die medizinische Vorrichtung.

**11.** Medizinisches System umfassend:

- eine Sprachsteuervorrichtung (10) nach Anspruch 10; und
- eine medizinische Vorrichtung (1) zur Durchführung einer medizinischen Prozedur.

**12.** Computerprogrammprodukt, welches ein Programm umfasst und direkt in einen Speicher einer programmierbaren Recheneinheit ladbar ist, mit Programmmitteln, um ein Verfahren nach den Ansprüchen 1 bis 9 auszuführen, wenn das Programm ausgeführt wird.

**13.** Computerlesbares Speichermedium, auf welchem lesbare und ausführbare Programmabschnitte gespeichert sind, um alle Schritte eines Verfahrens nach einem der Ansprüche 1 bis 9 auszuführen, wenn die Programmabschnitte ausgeführt werden.

**Claims**

**1.** Method for voice control of a medical apparatus (1) with the steps:

- capturing (S10) an audio signal (E1) containing operator voice input directed at controlling the apparatus;
- first analysis (S20) of the audio signal for providing a first voice analysis result (SAE1),
- recognising (S30) a first voice command (SSB1) based on the first voice analysis result,
- assigning (S40) the first voice command to a safety class (SK), wherein a safety class is provided for safety-critical voice commands,
- ascertaining (S50) a verification signal (VS) to confirm the first voice command when the first voice command has been assigned to a safety class for safety-critical speed commands,
- generating (S60) a control signal (C1) for controlling the medical apparatus based on the first voice command and the verification signal provided that the first voice command has been confirmed, wherein the control signal is suitable for controlling the medical apparatus according to the first voice command, and
- inputting (S70) the control signal into the medical apparatus
wherein
- the ascertaining of the verification signal comprises an outputting of the first voice command and a prompt (A1) to confirm the first voice command to the operator (S51-1) and a capturing (S51-2) of an operator user input (E2) directed at the confirmation of the first voice command,
- the verification signal confirms the first voice command if the operator user input directed at the confirmation of the first voice command satisfies
- a predefined time criterion (ZK) and
- the time criterion can be adjusted in dependence on the first voice command.

2. Method according to claim 1, wherein

- the outputting comprises outputting an audio signal (A1) based on the voice command, and/or
- the capturing comprises capturing user input (E2) embodied as an audio signal.

3. Method according to one of claims 1 to 2, wherein the verification signal confirms the first voice command if the operator user input directed at the confirmation of the first voice command satisfies

- a predefined content criterion (IK).

4. Method according to one of claims 1 to 3, wherein ascertaining the verification signal also comprises outputting a prompt (A2) for user input of voice control information (SSI) specific to the first voice command to the operator and capturing user input comprising the voice control information.

5. Method according to one of the preceding claims, wherein the analysis comprises applying a first computational linguistics algorithm comprising a first trained function to the audio signal.

6. Method according to one of the preceding claims, wherein ascertaining the verification signal comprises

- analysing (S52-1) the audio signal to provide a second voice analysis result (SAE2),
- recognising (S52-3) a second voice command (SSB2) based on the second voice analysis result,
- comparing (S52-4) the first and second voice command, wherein the verification signal confirms the first voice command if the first voice command and the second voice command satisfy a conformity criterion (ÜK).

7. Method according to claim 5 and 6, wherein the analysis comprises applying a second computational linguistics algorithm comprising a second trained function to the audio signal, wherein the first trained function and the second trained function are different from one another.

8. Method according to claim 7, wherein the second trained function is embodied only to identify safety-critical voice commands in the audio signal.

9. Method according to one of the preceding claims, wherein analysing the audio signal comprises

- tokenising for segmenting letters, words and/or sentences within the audio signal and the first and/or second voice command are recognised based on first tokenisation information and/or second tokenisation information, and/or
- semantic analysis of the audio signal and the first and second voice command are recognised based on first semantic information and second semantic information.

10. Voice control apparatus (10) for voice control of a medical apparatus (1) comprising

- at least one interface (31, 34) for capturing an audio signal (E1) containing operator voice input directed at controlling the apparatus;
- at least one evaluation unit (3, M1, M2, OM1) embodied

- to analyse the audio signal and to provide a first voice analysis result (SAE1),
- to recognise a first voice command (SSBI) based on the first voice analysis result,
- to assign the first voice command to a safety class (SK), wherein a safety class is provided for safety-critical voice commands,
- to ascertain a verification signal (VS) to confirm the first voice command, when the first voice command is assigned to a safety class for safety-critical voice commands, wherein

- the evaluation unit is embodied to output (S51-1) the first voice command and a prompt (A1) to confirm the first voice command to the operator and to capture an operator user input (E2) directed at the confirmation of the first voice command,
- the verification signal confirms the first voice command when the operator user input directed at the confirmation of the first voice command satisfies
- a predefined time criterion (ZK) and
- the time criterion can be adjusted in dependence on the first voice command, and

- a control unit (3, M3) embodied

- to generate a control signal (C1) for controlling the medical apparatus based on the first voice command and the verification signal provided that the first voice command has been confirmed based on the verification signal, wherein the control signal is suitable for controlling the medical apparatus according to the first voice command, and

- an interface (32) for inputting the control signal into the medical apparatus.

11. Medical system comprising:

- a voice control apparatus (10) according to claim 10; and
- a medical apparatus (1) for performing a medical procedure.

12. Computer program product, which comprises a program and can be loaded directly into a memory of a programmable computing unit, with program means for executing a method according to claims 1 to 9 when the program is executed.

13. Computer-readable storage medium on which readable and executable program sections are stored in order to execute all the steps of a method according to one of claims 1 to 9 when the program sections are executed.

**Revendications**

1. Procédé de commande vocale d'un dispositif (1) médical comprenant les stades :

- détection (S10) d'un signal (E1) audio contenant une entrée vocale, visant à la commande du dispositif, d'une personne de service ;
- première analyse (S20) du signal audio pour disposer d'un premier résultat (SAE1) d'analyse vocale,
- identification (S30) d'une première instruction (SSB1) vocale sur la base du premier résultat d'analyse vocale,
- affectation (S40) de la première instruction vocale à une classe (SK) de sécurité, dans lequel une classe de sécurité est prévue pour des instructions vocales critiques du point de vue de la sécurité,
- détermination (S50) d'un signal (VS) de vérification, pour la confirmation de la première instruction vocale, si la première instruction vocale a été affectée à une classe de sécurité pour des instructions vocales critiques du point de vue de la sécurité,
- production (S60) d'un signal (C1) de commande pour la commande du dispositif médical sur la base de la première instruction vocale et du signal de vérification, pour autant que la première instruction vocale a été confirmée, dans lequel le signal de commande est propre à commander le dispositif médical conformément à la première instruction vocale, et
- entrée (S70) du signal de commande dans le dispositif médical, dans lequel
- la détermination du signal de vérification comprend une sortie de la première instruction vocale et d'une invitation (A1) à la confirmation de la première instruction vocale à la personne (S51-1) de service ainsi qu'une détection (S51-2) d'une entrée (E2) d'utilisateur, visant à la confirmation de la première instruction vocale, de la personne de service
- le signal de vérification confirme la première instruction vocale si l'entrée d'utilisateur, visant à la confirmation de la première instruction vocale, de la personne de service,
- satisfait un critère (ZK) temporel défini à l'avance, et
- le critère temporel peut, en fonction de la première instruction vocale, être adapté suivant des prescriptions spécifiques d'utilisateur.

2. Procédé suivant la revendication 1, dans lequel

- la sortie comprend une sortie d'un signal (A1) audio reposant sur l'instruction vocale, et/ou
- la détection comprend une détection d'une entrée (E2) d'utilisateur constituée sous la forme d'un signal audio.

3. Procédé suivant l'une des revendications 1 à 2, dans lequel le signal de vérification confirme la première instruction vocale si l'entrée d'utilisateur, visant à la confirmation de la première instruction vocale, de la personne de service

   - satisfait un critère (IK) de contenu défini à l'avance.

4. Procédé suivant l'une des revendications 1 à 3, dans lequel la détermination du signal de vérification comprend également une invitation (A2) pour l'entrée d'utilisateur à donner à la personne de service une information (SSI) de commande vocale spécifique à la première instruction vocale et à détecter une entrée d'utilisateur comprenant l'information de commande vocale.

5. Procédé suivant l'une des revendications précédentes, dans lequel l'analyse comprend une application d'un premier algorithme linguistique informatique comprenant une première fonction entraînée sur le signal audio.

6. Procédé suivant l'une des revendications précédentes, dans lequel la détermination du signal de vérification comprend

   - analyse (S52-1) du signal audio pour disposer d'un deuxième résultat (SAE2) d'analyse vocale,
   - identification (S52-3) d'une deuxième instruction (SSB2) vocale sur la base du deuxième résultat d'analyse vocale,
   - comparaison (S52-4) de la première et de la deuxième instructions vocales,

   dans lequel le signal de vérification confirme la première instruction vocale si la première instruction vocale et la deuxième instruction vocale satisfont un critère (ÜK) de concordance.

7. Procédé suivant la revendication 5 et 6, dans lequel l'analyse comprend une application d'un deuxième algorithme linguistique informatique comprenant une deuxième fonction entraînée et le signal audio, dans lequel la première fonction entraînée et la deuxième fonction entraînée se distinguent l'une de l'autre.

8. Procédé suivant la revendication 7, dans lequel la deuxième fonction entraînée est constituée pour n'identifier, dans le signal audio, que des instructions vocales critiques du point de vue de la sécurité.

9. Procédé suivant l'une des revendications précédentes, dans lequel l'analyse du signal audio

   - comprend un procédé au jeton de segmentation de lettres, de mots et/ou de phrases dans le signal audio et la première et/ou la deuxième instructions vocales sont identifiées sur la base d'une première information de procédé au jeton et/ou d'une deuxième information de procédé au jeton,
   et/ou
   - comprend une analyse sémantique du signal audio et la première et la deuxième instructions vocales sont identifiées sur la base d'une première information sémantique et d'une deuxième information sémantique.

10. Dispositif (10) de commande vocale pour la commande vocale d'un dispositif (1) médical, comprenant

   - au moins une interface (31, 34) de détection d'un signal (E1) audio contenant une entrée vocale, visant à la commande du dispositif, d'une personne de service ;
   - au moins une unité (3, M1, M2, OM1) d'évaluation, qui est constituée

      - pour analyser le signal audio et disposer d'un premier résultat (SAE1) d'analyse vocale,
      - pour identifier une première instruction (SSB1) vocale sur la base du premier résultat d'analyse vocale,
      - pour affecter la première instruction vocale à une classe (SK) de sécurité, dans lequel une classe de sécurité est prévue pour des instructions vocales critiques du point de vue de la sécurité,
      - pour déterminer un signal (VS) de vérification pour la confirmation de la première instruction vocale, si la première instruction vocale a été affectée à une classe de sécurité pour des instructions vocales critiques du point de vue de la sécurité,
      dans lequel
      - l'unité d'évaluation est constituée pour donner (S51-1) à la personne de service la première instruction vocale et une invitation (A1) à la confirmation de la première instruction vocale et pour détecter une entrée (E2) d'utilisateur, visant à la confirmation de la première instruction vocale, de la personne de service,
      - le signal de vérification confirme la première instruction vocale si l'entrée d'utilisateur, visant à la confirmation de la première instruction vocale, de la personne de service,

- satisfait un critère (ZK) temporel défini à l'avance, et
- le critère temporel peut, en fonction de la première instruction vocale, être adapté suivant des prescriptions spécifiques d'utilisateur, et

- une unité (3, M3) de commande, qui est constituée

- pour produire un signal (C1) de commande pour la commande du dispositif médical sur la base de la première instruction vocale et du signal de vérification, pour autant que la première instruction vocale a été confirmée à l'aide du signal de vérification, dans lequel le signal de commande est propre à commander le dispositif médical conformément à la première instruction vocale, et

- une interface (32) pour l'entrée du signal de commande dans le dispositif médical.

11. Système médical comprenant :

- un dispositif (10) de commande vocale suivant la revendication 10 ; et
- un dispositif (1) médical pour effectuer une procédure médicale.

12. Produit de programme d'ordinateur, qui comprend un programme et qui peut être chargé directement dans la mémoire d'une unité informatique programmable, comprenant des moyens de programme, pour exécuter un procédé suivant l'une des revendications 1 à 9, lorsque le programme est exécuté.

13. Support de mémoire déchiffrable par ordinateur, sur lequel sont mises en mémoire des parties de programme déchiffrables et exécutables, afin d'exécuter tous les stades d'un procédé suivant l'une des revendications 1 à 9, lorsque les parties de programme sont exécutées.

FIG 1

FIG 2

# FIG 3

S10

— E1

S20

— SAE1

S30

— SSB1

S40

— SK

S50

— VS

S60

— C1

S70

# FIG 4

S51-1/S51-11

— A1/A2

S51-2

— E2/SSI

S51-3

— IK/ZK

S51-4

ΔZK

S51-5

VS

# FIG 5

FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006045719 B4
- US 20140195247 A1
- US 9824689 B1